# FASCICULE DE BREVET EUROPEEN

(11) **EP 0 894 796 B1**
(45) Date de publication et mention de la délivrance du brevet: **06.06.2001**
(21) Numéro de dépôt: 98401919.0
(22) Date de dépôt: 28.07.1998
(51) Int. Cl.: C07D 413/04, C07D 413/06, C07D 498/06, A61K 31/535, A61K 31/54

(54) **Imidazolines substituées par un noyau hétérocyclique, alpha-2 antaginistes**
Durch einen heterocyclischen Ring substituierte Imidazoline als alpha-2 Antagonisten
Imidazolines substituted with a heterocyclic ring as alpha-2 antagonists

(30) Priorité: 30.07.1997 FR 9709709
(43) Date de publication de la demande: 03.02.1999
(73) Titulaire: ADIR ET COMPAGNIE, 92415 Courbevoie Cédex (FR)
(72) Inventeur: Guillaumet, Gérald, 45650 Saint Jean le Blanc (FR); Merour, Jean-Yves, 45160 Olivet (FR); Touzeau, Frédérique, 45160 Olivet (FR); Pfeiffer, Bruno, 95320 Saint Leu la Forêt (FR); Renard, Pierre, 78000 Versailles (FR); Scalbert, Elisabeth, 92100 Boulogne (FR)

(56) Documents cités:
- EP-A- 0 058 006
- EP-A- 0 074 711
- EP-A- 0 092 328
- R.C.M. BUTLER ET AL.: "Synthesis of 2-(2-imidazolinyl) substituted 2,3-dihydro-4H-1,4-benzothiazine and 3,4-dihydro-2H-1,4-benzoxazines" JOURNAL OF HETEROCYCLIC CHEMISTRY., vol. 22, no. 1, 1985, pages 178-181, XP002061221 PROVO US
- Michel, M. C., Ernsberger, P.; 'Keeping an eye on the I site: imidazoline-preferring receptors' Trends Pharmacol. Sci. 1992, 253, 408

## Description

L'invention concerne de nouveaux dérivés hétérocycliques, leur procédé de préparation et les compositions pharmaceutiques qui les contiennent.

Ces nouveaux composés se caractérisent, d'un point de vue structural par la présence d'un noyau imidazoline substitué par un motif hétérocyclique. Certains dérivés de ce type ont été décrits dans la littérature pour leurs propriétés α₂ antagonistes et pour leur utilité dans le traitement de la dépression (R.C.M. Butler et al., J. Heterocyclic. Chem., 1985, *22*, 177 ; EP 92328 ; EP 58006 ; EP 74711).

Par contre, les dérivés de la présente invention possèdent une très grande affinité pour les récepteurs aux imidazolines, ce qui est particulièrement intéressant pour la mise au point de nouvelles substances pharmacologiquement actives.

Récemment, ont été mis en évidence, au niveau du système nerveux central, des sites de liaison spécifique des composés de structure imidazoline (P. Bousquet et al., Biochem. Pharmacol., 1983, *32*, 1459 ; J. Pharmacol. Exp. Ther., 1984, *230*, 232), différents des récepteurs de l'histamine (P. Emsberger et al. Soc. Neurosci. Abstr., 1986, *12*, 1334). Ces récepteurs ont été caractérisés au niveau du *nucleus* reticularis *lateralis*, dans la partie rostroventrale du bulbe rachidien (G. Brica et al., Eur. J. Pharmacol., 1989, *162,* 1), sont distribués de façon hétérogène dans le cerveau (P. Emsberger et al., J. Pharmacol. Exp. Ther., 1990, *253*, 408), et sont également présents au niveau périphérique (P. Ernsberger et al., Am. J. Hypertens., 1990, *3*, 90 ; J. E. Piletz et al., Biochem. Pharmacol., 1991, *42*, 346). Deux sous-types de récepteurs I₁ et I₂ ont été identifiés (M.C. Michel et al., Trends Pharmacol. Sci., 1992, *13*, 369) en fonction de leur capacité à lier la Clonidine (sous type I₁) et l'Idazoxan (sous type I₂).

Ces récepteurs semblent être fortement impliqués aux côtés des adrénorécepteurs α₂ dans l'effet vasodépressif et antihypertenseur de certains composés comme la Clonidine et la Rilmenidine (G. Brica et al. Eur. J. Pharmacol., 1989, *162*, 1 ; R.E. Gomez et al. Eur. J. Pharmacol., 1991, *195*, 181). D'autre part, leur rôle dans la stimulation de la libération d'insuline par les cellules β du pancréas a été démontré (Schutz et al., Naunyn-Schniedeberg's Arch. Pharmacol., 1989, *340*, (6/712). Il a également été démontré que des ligands des récepteurs aux imidazolines peuvent avoir un intérêt particulier dans le traitement des désordres psychiatriques et neurologiques (D.S. Nutt et al., Annals New York Academy of Sciences, 1995, 125).
Les composés de l'invention présentent une structure originale qui leur confère de façon surprenante une très grande affinité pour les récepteurs aux imidazolines. Ils sont donc susceptibles d'être utilisés pour le traitement de pathologies liées à des dérèglements du fonctionnement de ces récepteurs, comme les maladies cardio-vasculaires et l'hypertension artérielle, le diabète, l'obésité, les désordres psychiatriques et neurologiques comme la dépression, la maladie de Parkinson, l'anorexie , la maladie d'Alzheimer, etc...

De façon préférentielle, les composés de l'invention seront utilisés dans le traitement des maladies cardiovasculaires et de l'hypertension artérielle.

Plus spécifiquement, la présente invention concerne les composés de formule (I) : 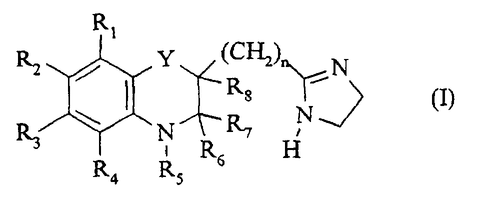 dans laquelle :
- n est égal à 0 ou 1,
- Y représente un atome d'oxygène, de soufre, ou un groupement CH₂,
- R₁, R₂, R₃, R₄, identiques ou différents, représentent un atome d'hydrogène, d'halogène, un groupement alkyle (C₁-C₆) linéaire ou ramifié, cycloalkyle (C₃-C₇), alkoxy (C₁-C₆) linéaire ou ramifié, hydroxy, hydroxyalkyle (C₁-C₆) linéaire ou ramifié, trihalogénoalkyle (C₁-C₆) linéaire ou ramifié, alkyle (C₁-C₆) carbonyle linéaire ou ramifié, formyle, cyano, carboxy, alkoxy (C₁-C₆) carbonyle linéaire ou ramifié, nitro, amino éventuellement substitué, ou bien, (R₁-R₂) ou (R₂-R₃) ou (R₃-R₄) forment avec les carbones auxquels ils sont liés un cycle à 5 ou 6 chaînons saturé ou insaturé éventuellement substitué,
- R₅ représente un atome d'hydrogène, un groupement cycloalkyle (C₃-C₇), ou un groupement alkyle (C₁-C₆) linéaire ou ramifié éventuellement substitué par un groupement choisi parmi cycloalkyle (C₃-C₇) et phényle éventuellement substitué, ou forme avec R₄ un cycle à 5, 6 ou 7 chaînons saturé ou insaturé, éventuellement substitué par un ou plusieurs groupements, identiques ou différents, choisis parmi alkyle (C₁-C₆) linéaire ou ramifié, alkoxy (C₁-C₆) linéaire ou ramifié, hydroxy, oxo et amino éventuellement substitué,
- R₆ et R₇, représentent chacun un atome d'hydrogène ou forment ensemble un groupement oxo,
- R₈ représente un atome d'halogène, d'hydrogène ou un groupement alkyle (C₁-C₆) linéaire ou ramifié, alkényle (C₂-C₆) linéaire ou ramifié, hydroxy, alkoxy (C₁-C₆) linéaire ou ramifié (éventuellement substitué par un groupement amino, pyrolyle, pipéridinyle), cycloalkyloxy (C₃-C₇), phényloxy éventuellement substitué, benzyloxy éventuellement substitué, ou forme avec R₇ une liaison,
étant entendu que :
- lorsque n est égal à 0, Y représente un atome d'oxygène et R₁, R₂, R₃, R₄, R₆, R₇, R₈ représentent chacun un atome d'hydrogène, alors R₅ est différent d'un atome d'hydrogène, d'un groupement méthyle, éthyle ou benzyle,
- lorsque n est égal à 0, Y représente un atome de soufre et R₁, R₂, R₃, R₄, R₆, R₇, R₈ représentent chacun un atome d'hydrogène, alors R₅ est différent d'un atome d'hydrogène,
leurs N-oxydes, leurs énantiomères, diastéréoisomères, ainsi que leurs sels d'addition à un' acide ou à une base pharmaceutiquement acceptable.

Par groupement amino éventuellement substitué, on entend substitué par un ou deux groupements, identiques ou différents, choisis parmi alkyle (C₁-C₆) linéaire ou ramifié, phényle éventuellement substitué, benzyle éventuellement substitué.

Les termes éventuellement substitué, affectés aux groupements phényloxy, benzyloxy, phényle et benzyle ainsi qu'à l'expression "cycle à 5 ou 6 chaînons", signifient que ces groupements peuvent être substitués par un ou plusieurs substituants, identiques ou différents, choisis parmi halogène, alkyle (C₁-C₆) linéaire ou ramifié, alkoxy (C₁-C₆) linéaire ou ramifié, hydroxy et trihalogénoalkyle (C₁-C₆) linéaire ou ramifié.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthanesulfonique, camphorique, oxalique etc...

Parmi les bases pharmaceutiquement acceptables, on peut citer à titre non limitatif l'hydroxyde de sodium, l'hydroxyde de potassium, la triéthylamine, la tertbutylamine, etc...

L'invention concerne préférentiellement les composés de formule (I) pour lesquels n est égal à 0.

Les composés préférés de l'invention sont ceux pour lesquels Y représente un atome d'oxygène. D'autres composés préférés de l'invention sont ceux pour lesquels Y représente un atome de soufre.

Dans les composés de formule (I), R₆, R₇ et R₈ représentent préférentiellement chacun un atome d'hydrogène.

De façon préférentielle dans les composés de formule (I), trois des groupements R₁, R₂, R₃ et R₄ sont identiques et représentent chacun un atome d'hydrogène, le groupement restant étant tel que défini dans la formule (I).

D'autres composés préférés de formule (I) sont ceux pour lesquels (R₁-R₂) ou (R₂-R₃) ou (R₃-R₄) forment avec les carbones auxquels ils sont liés un cycle à 5 ou 6 chaînons saturé ou insaturé éventuellement substitué, par exemple un cycle phényle ou cyclohexyle.

Dans les composés de formule (I), un groupement préféré pour R₈ est le groupement alkyle (C₁-C₆) linéaire ou ramifié.

Dans les composés de formule (I), un groupement préféré pour R₅ est le groupement alkyle (C₁-C₆) linéaire ou ramifié.

Préférentiellement dans les composés de formule (I), R₄ et R₅ forment ensemble un cycle à 5, 6 ou 7 chaînons, saturé ou insaturé, éventuellement substitué.

Plus préférentiellement, l'invention concerne les composés de formule (I) pour lesquels Y représente un atome d'oxygène, n vaut 0, R₆, R₇ et R₈ représentent chacun un atome d'hydrogène, R₁, R₂, R₃, R₄, identiques ou différents, représentent un atome d'hydrogène, d'halogène, un groupement alkyle (C₁-C₆) linéaire ou ramifié, cycloalkyle (C₃-C₇), alkoxy (C₁-C₆) linéaire ou ramifié, hydroxy, hydroxyalkyle (C₁-C₆) linéaire ou ramifié, trihalogénoalkyle (C₁-C₆) linéaire ou ramifié, alkyle (C₁-C₆) carbonyle linéaire ou ramifié, formyle, cyano, carboxy, alkoxy (C₁-C₆) carbonyle linéaire ou ramifié, nitro, amino éventuellement substitué, ou bien, (R₁-R₂) ou (R₂-R₃) ou (R₃-R₄) forment avec les carbones auxquels ils sont liés un cycle à 5 ou 6 chaînons saturé ou insaturé éventuellement substitué, tandis que R₅ représente un groupement alkyle (C₁-C₆) linéaire ou ramifié ou forme avec R₄ un cycle à 5, 6 ou 7 chaînons, saturé ou insaturé.

L'invention s'étend également au procédé de préparation des composés de formule (I) caractérisé :
- lorsque dans le composé de formule (I) que l'on souhaite obtenir, R₄ et R₅ ne forment pas ensemble un cycle, en ce que l'on utilise comme matière première un composé de formule (II/a) : 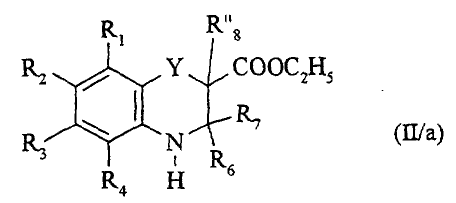 dans laquelle R"₈ représente un atome d'hydrogène, ou forme avec R₇ une liaison et Y, R₁, R₂, R₃, R₄, R₆ et R₇ sont tels que définis dans la formule (I),
que l'on traite,
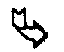 soit par un dérivé de formule (III):

   R'₅-X (III)

   dans laquelle X représente un atome d'halogène et R'₅ représente un groupement cycloalkyle (C₃-C₇), ou un groupement alkyle (C₁-C₆) linéaire ou ramifié éventuellement substitué par un groupement cycloalkyle (C₃-C₇) , phényle éventuellement substitué,
   pour conduire à un composé de formule (II/b) : 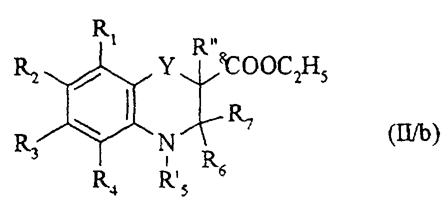 dans laquelle Y, R₁, R₂, R₃, R₄, R'₅, R₆, R₇ et R"₈ sont tels que définis précédemment,
   qui peut être soumis, lorsque R"₈ représente un atome d'hydrogène, à l'action d'un agent d'halogénation, ou bien à l'action d'une base forte suivie d'un traitement par un dérivé de formule (IV):

   R'₈-X (IV)

   dans laquelle X représente un atome d'halogène et R'₈, différent d'un atome d'hydrogène, a la même signification que R₈ dans la formule (I),
   pour conduire à un composé de formule (II/c) : 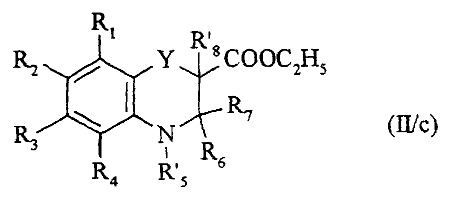 dans laquelle Y, R₁, R₂, R₃, R₄, R'₅, R₆ et R₇ et R'₈ sont tels que définis précédemment,
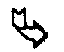 soit, lorsque dans le composé de formule (II/a) utilisé, R"₈ représente un atome d'hydrogène, par un dérivé de formule (IV) telle que définie précédemment, après un traitement par une base forte,
   pour conduire à un composé de formule (II/d) : 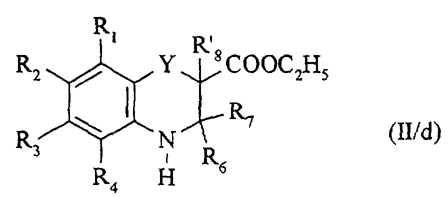 dans laquelle Y, R₁, R₂, R₃, R₄, R₆ R₇ et R'₈ sont tels que définis précédemment,
   composés (II/a), (II/b), (II/c) et (II/d), formant la totalité des composés de formule (II) : 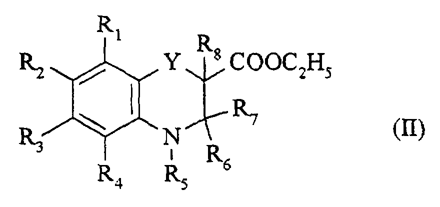 dans laquelle Y, R₁, R₂, R₃, R₄, R₅, R₆, R₇ et R₈ sont tels que définis précédemment,
   que l'on traite, éventuellement après homologation d'un carbone de la chaine portant la fonction ester, par l'éthylènediamine pour conduire à un composé de formule (I/a) : 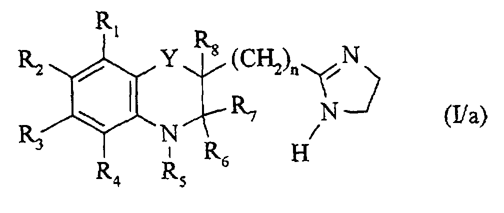 cas particulier des composés de formule I, pour lequel, Y, R₁, R₂, R₃, R₄, R₅, R₆, R₇, R₈ et n ont la même signification que dans la formule (I), étant entendu que R₄ et R₅ ne forment pas ensemble un cycle,
ou bien,
- lorsque dans le composé de formule (I) que l'on souhaite obtenir, R₄ et R₅ forment ensemble un cycle, en ce que l'on utilise comme matière première un composé de formule (II/e) : 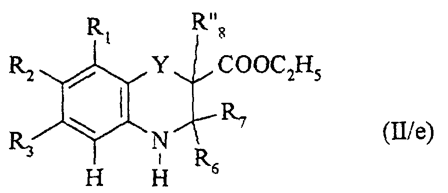 dans laquelle R₁, R₂, R₃, R₆, R₇ et R"₈ sont tels que définis précédemment,
   que l'on traite par un dérivé de formule (V) ou (VI) : 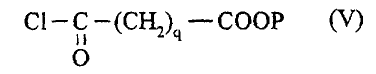 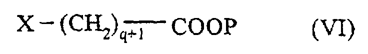 dans lesquelles X représente un atome d'halogène ou un groupe partant, P un groupement masquant la fonction acide, et q un entier égal à 0, 1 ou 2,
   pour conduire à un composé de formule (VII) : 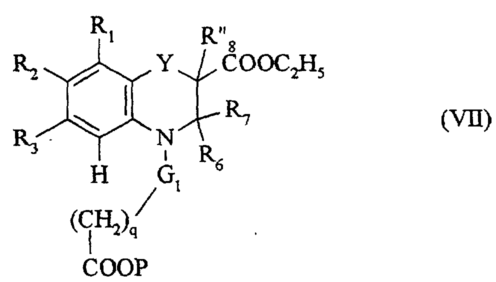 dans laquelle G₁ représente un groupement CH₂ ou CO, et q, P, Y, R₁, R₂, R₃, R₆, R₇ et R"₈ ont la même signification que précédemment,
   qui, après libération de la fonction acide et cyclisation, conduit au composé de formule (VIII/a) : 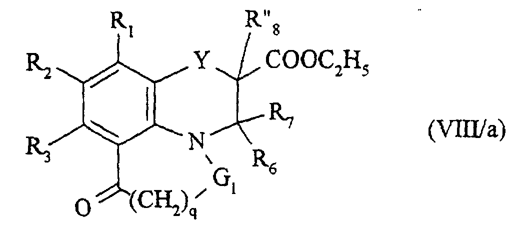 dans laquelle Y, R₁, R₂, R₃, R₆, R₇, R"₈, G₁ et q ont la même signification que précédemment,
composé (VIII/a),
- qui peut être transformé, après traitement par un halogénure d'alkyle, en un composé de formule (VIII/b): 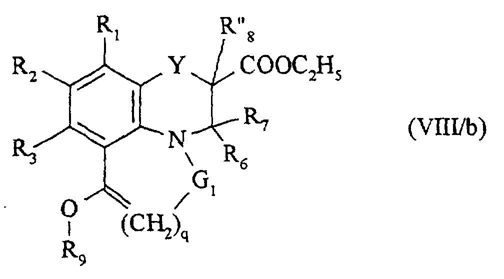 dans laquelle R₉ représente un groupement alkyle (C₁-C₆) linéaire ou ramifié, et Y, R₁, R₂, R₃, R₆, R₇, R"₈, G₁, et q ont la même signification que précédemment,
- ou peut être réduit pour conduire à un composé de formule (VIII/c) : 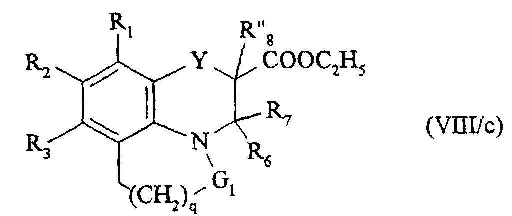 dans laquelle Y, R₁, R₂, R₃, R₆, R₇, R"₈, G₁ et q sont tels que définis précédemment,
chacun des composés (VIII/a), (VIII/b) et (VIII/c) pouvant être traité, lorsque cela est compatible avec les substituants présents sur la molécule, par une base forte, puis par un dérivé de formule (IV) telle que définie précédemment, pour conduire au composé de formule (VIII/d) : 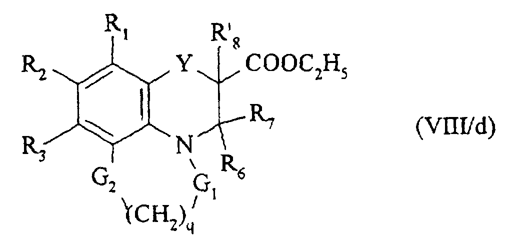 dans laquelle G₂ représente un groupement CH₂, CO, ou forme avec le carbone du méthylène adjacent un groupement 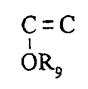 et Y, R₁, R₂, R₃, R₆, R₇, R'₈, R₉, G₁ et q sont tels que définis précédemment,
composés (VIII/a), (VIII/b), (VIII/c), et (VIII/d) formant la totalité des composés de formule (VIII): 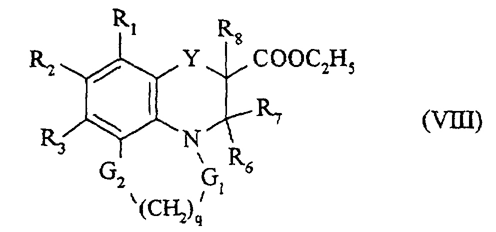 dans laquelle R₁, R₂, R₃, R₆, R₇, R₈, G₁, G₂ et q sont tels que définis précédemment,
et qui sont traités, éventuellement après homologation d'un carbone de la chaîne portant la fonction ester, par l'éthylènediamine pour conduire à un composé de formule (I/b) : 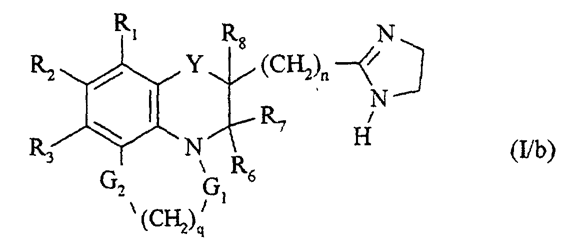 cas particulier des composés de formule (I), pour lequel Y, R₁, R₂, R₃, R₆, R₇, R₈, G₁, G₂, n et q sont tels que définis précédemment,
composés (I/a) et (I/b) :
- que l'on purifie éventuellement selon une technique classique de purification,
- dont on sépare éventuellement les isomères selon une technique classique de séparation,
- et que l'on transforme, le cas échéant, en leurs N-oxydes ou en leurs sels d'addition à un acide ou à une base pharmaceutiquement acceptable.

Dans le but de réaliser une synthèse plus adaptée à certains produits de formule (I) que l'on souhaite obtenir, certaines variantes du procédé indiqué ci-dessus pourront être utilisées.

L'une d'entre elle consiste à utiliser comme matière première le dérivé de formule (IX) : 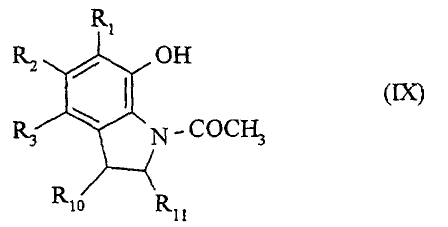 dans laquelle R₁₀ et R₁₁ représentent simultanément un hydrogène ou forment ensemble une liaison, R₁, R₂, R₃ ayant la même signification que dans la formule (I),
qui, après déprotection de l'azote indolique, est traité par le 2,3-dibromopropionate d'éthyle en milieu basique, pour conduire au composé de formule (X/a) : 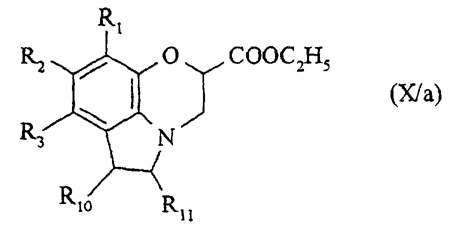 dans laquelle R₁, R₂, R₃, R₁₀, R₁₁ sont tels que définis précédemment,
que l'on peut faire réagir avec une base forte et un dérivé de formule (IV) :

R'₈ - X (IV)

dans laquelle X représente un atome d'halogène et R'₈, différent d'un atome d'hydrogène, a la même signification que R₈ dans la formule (I),
pour conduire au composé de formule (X/b) : 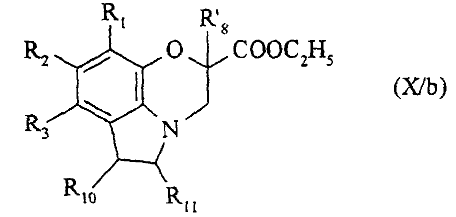 dans laquelle R₁, R₂, R₃, R'₈, R₁₀, R₁₁ sont tels que définis précédemment,
composés (X/a) et (X/b) qui représentent la totalité des composés de formule (X): 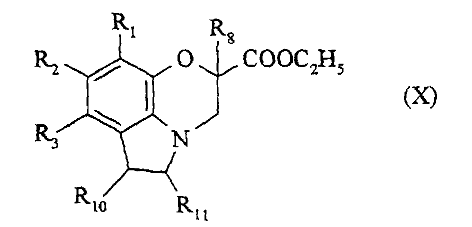 dans laquelle R₁, R₂, R₃, R₈, R₁₀ et R₁₁ sont tels que définis précédemment,
que l'on traite, après homologation éventuelle d'un carbone de la chaîne portant la fonction ester, par l'éthylènediamine, pour conduire au composé de formule (I/c) : 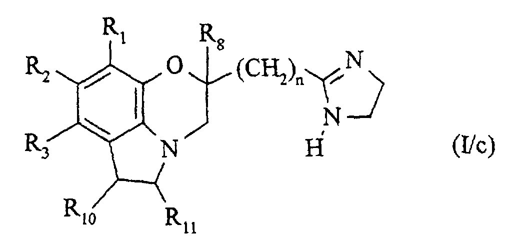 cas particulier des composés de formule (I) pour lequel R₁, R₂, R₃, R₈, R₁₀, R₁₁ et n sont tels que définis précédemment,
composé (I/c)
- que l'on purifie éventuellement selon une technique classique de purification,
- dont on sépare eventuellement les isomères selon une technique classique de séparation,
- et que l'on transforme, le cas échéant, en son N-oxyde ou en son sel d'addition à un acide ou à une base pharmaceutiquement acceptable,
étant entendu que lorsque R₁₀ et R₁₁ représentent chacun un atome d'hydrogène, ils peuvent être transformés en une liaison par une méthode classique d'aromatisation, à tout moment du procédé.

L'invention s'étend également aux compositions pharmaceutiques renfermant comme principe actif au moins un composé de formule (I) seul ou en combinaison avec un ou plusieurs excipients ou véhicules inertes non toxiques.

Parmi les compositions pharmaceutiques selon l'invention on pourra citer plus particulièrement celles qui conviennent pour l'administration orale, parentérale, nasale, les comprimés simples ou dragéifiés, les comprimés sublinguaux, les gélules, les tablettes, les suppositoires, les crèmes, pommades, gels dermiques, etc...

La posologie utile varie selon l'âge et le poids du patient, la nature et la sévérité de l'affection ainsi que la voie d'administration. Celle-ci peut être nasale, rectale, parentérale ou orale. D'une manière générale, la posologie unitaire s'échelonne entre 1 et 500 mg pour un traitement en 1 à 3 prises par 24 heures.

Les préparations suivantes (Préparations A-D) conduisent à des intermédiaires de synthèse utiles dans la préparation des composés de l'invention.

### Préparation A : 2-Ethoxycarbonyl-6-fluoro-2,3-dihydro-2H-1,4-benzoxazine

### Stade a : 4-Fluoro-2-aminophénol

A une solution de 7,96 mmol (1,26 g) de 4-fluoro-2-nitrophénol dans 20 ml d'éthanol sont ajoutées 47,7 mmol (10,8 g) de SnCl₂,H₂O. La solution est agitée à reflux durant 2 heures. Après refroidissement, le milieu réactionnel est hydrolysé sur de la glace, et alcalinisé par une solution de soude à 30 %. Le milieu est extrait 4 fois par l'acétate d'éthyle, et les phases organiques regroupées sont lavées par une solution aqueuse saturée de chlorure de sodium, puis séchée sur sulfate de magnésium et concentrées pour conduire au produit attendu.

### Stade b : 2-Ethoxycarbonyl-6-fluoro-2,3-dihydro-2H-1,4-benzoxazine

Le produit attendu est obtenu selon le procédé décrit dans J. Heterocyclic. Chem., 1985, *22*, 177, à partir du composé décrit au stade précédent.

### Préparation B : 2-Ethoxycarbonyl-6-trifluorométhyl-3,4-dihydro-2H-1,4-benzoxazine

Le produit attendu est obtenu selon le procédé décrit dans la préparation A en utilisant comme produit de départ le 4-trifluorométhyl-2-nitrophénol.

### Préparation C : 2-Ethoxycarbonyl-8-trifluorométhyl-3,4-dihydro-2H-1,4-benzoxazine

Le produit attendu est obtenu selon le procédé décrit dans la préparation A en utilisant comme produit de départ le 6-trifluorométhyl-2-nitrophénol.

### Préparation D : 2-Ethoxycarbonyl-7-méthoxy-3,4-dihydro-2H-1,4-benzoxazine

Le produit attendu est obtenu selon le procédé décrit dans la préparation A en utilisant comme produit de départ le 4-méthoxy-2-nitrophénol.

Les exemples suivants illustrent l'invention et ne la limitent en aucune façon. Les structures des composés décrits ont été confirmées par les techniques spectroscopiques usuelles

### EXEMPLE 1 : 2-(4,5-Dihydro-1H-2-imidazolyl)-4-propyl-3,4-dihydro-2H-1,4-benzoxazine, oxalate

### Stade a : 2-Ethoxycarbonyl-4-propyl-2,3-dihydro-2H-1,4-benzoxazine

A 9,2 mmol (1,92 g) de 2-éthoxycarbonyl-3,4-dihydro-2*H*-1,4-benzoxazine (décrite dans J. Heterocyclic Chem., 1985, *22*, 177) dans 20 ml d'acétonitrile et 2 ml d'hexaméthylphosphoramide sont ajoutées 27,8 mmol (3,84 g) de carbonate de potassium et 18,5 mmol (1,8 ml) d'iodopropane. Le milieu réactionnel est porté à reflux pendant 18 heures. Après refroidissement et filtration, le filtrat est évaporé, repris dans un mélange eau/acétate d'éthyle, et extrait. La phase organique est séchée, concentrée, et purifiée par chromatographie sur gel de silice en utilisant comme éluant un mélange acétate d'éthyle/éther de pétrole, 1/9, pour conduire au composé attendu.

### Stade b : 2-(4,5-Dihydro-1H-2-imidazolyl)-4-propyl-3,4-dihydro-2H-1,4-benzoxazine

A 18,8 mmol (9,4 ml) de triméthylaluminium 2*M* dans le toluène est ajoutée une solution de 18,8 mmol (1,13 g) d'éthylènediamine dans 50 ml de toluène, en maintenant la température inférieure à 10°C. Une solution de 11 mmol du composé décrit au stade a dans 10 ml de toluène est ajoutée et le milieu réactionnel est porté à reflux pendant 3 heures. Après refroidissement, on ajoute un mélange eau/méthanol/dichlorométhane (1/1/1), puis le milieu est porté à reflux 15 minutes supplémentaires. Après refroidissement et filtration sur célite, le filtrat est concentré. Le résidu est repris par un mélange eau/dichlorométhane, et extrait. La phase organique est séchée, concentrée et purifiée par chromatographie sur gel de silice en utilisant comme éluant un mélange dichlorométhane/méthanol/triéthylamine, 98/2/1, pour conduire au composé du titre. L'oxalate correspondant est obtenu par action d'une solution titrée d'acide oxalique dans l'éthanol.
*Point de fusion (base) : 138-140°C*

| Microanalyse élémentaire (oxalate) : | | | |
|---|---|---|---|
| | **C** | **H** | **N** |
| % calculé | 57,29 | 6,32 | 12,53 |
| % Trouvé | 57,39 | 6,33 | 12,39 |

### EXEMPLE 2 : 2-(4,5-Dihydro-1H-2-imidazolyl)-4,6-diméthyl-3,4-dihydro-2H-1,4-benzoxazine, oxalate

### Stade a : 2-Ethoxycarbonyl-4,6-diméthyl-3,4-dihydro-2H-1,4-benzoxazine

Le produit attendu est obtenu selon le procédé décrit dans l'exemple 1, stade a, en remplaçant l'iodopropane par l'iodométhane et en utilisant comme produit de départ la 2-éthoxycarbonyl-6-méthyl-3,4-dihydro-2*H*-1,4-benzoxazine (préparée selon le procédé décrit dans J. Heterocyclic Chem., 1985, *22*, 177, à partir de la 2-hydroxy-5-méthylaniline).

### Stade b : 2-(4,5-Dihydro-1H-2-imidazolyl)-4,6-diméthyl-3,4-dihydro-2H-1,4-benzoxazine, oxolate

Le produit attendu est obtenu selon le procédé décrit dans l'exemple 1, stade b, à partir du composé décrit au stade précédent.

| Microanalyse élémentaire : | | | |
|---|---|---|---|
| | **C** | **H** | **N** |
| % calculé | 56,07 | 5,96 | 13,08 |
| % Trouvé | 55,86 | 5,89 | 12,80 |

### EXEMPLE 3 : 2-(4,5-Dihydro-1H-2-imidazolyl)-4,5-diméthyl-3,4-dihydro-2H-1,4-benzoxazine, oxalate

### Stade a : 4,5-Diméthyl-2-éthoxycarbonyl-3,4-dihydro-2H-1,4-benzoxazine

Le produit attendu est obtenu selon le procédé décrit dans l'exemple 1, stade a, en remplaçant l'iodopropane par l'iodométhane et en utilisant comme produit de départ la 2-éthoxycarbonyl-5-méthyl-3,4-dihydro-2*H*-1,4-benzoxazine (préparée selon le procédé décrit dans J. Heterocyclic Chem., 1985, *22*, 177, à partir de la 2-hydroxy-4-méthylaniline).

### Stade b : 2-(4,5-Dihydro-1H-2-imidazolyl)-4,5-diméthyl-3,4-dihydro-2H-1,4-benzoxazine, oxalate

Le produit attendu est obtenu selon le procédé décrit dans l'exemple 1, stade b, à partir du composé décrit au stade précédent.
*Point de fusion (base) : 132-134°C*

| Microanalyse élémentaire : | | | |
|---|---|---|---|
| | **C** | **H** | **N** |
| % calculé | 56,07 | 5,96 | 13,08 |
| % Trouvé | 56,07 | 5,98 | 12,98 |

### EXEMPLE 4: 6-Chloro-2-(4,5-dihydro-1H-2-imidazolyl)-4-méthyl-3,4-dihydro-2H-1,4- benzoxazine, oxalate

### Stade a : 6-Chloro-2-éthoxycarbonyl-4-méthyl-2H-1,4-benzoxazine

Le produit attendu est obtenu selon le procédé décrit dans l'exemple 1, stade a, en remplaçant l'iodopropane par l'iodométhane et en utilisant comme produit de départ la 6-chloro-2-éthoxycarbonyl-3,4-dihydro-*2H*-1,4-benzoxazine (préparée selon le procédé décrit dans J. Heterocyclic Chem., 1985, *22*, 177, à partir de la 2-hydroxy-4-chloroaniline).

### Stade b : 6-Chloro-2-(4,5-dihydro-1H-2-imidazolyl)-4-méthyl-3,4-dihydro-2H-1,4-benzoxazine, oxalate

Le produit attendu est obtenu selon le procédé décrit dans l'exemple 1, stade b, à partir du composé décrit au stade précédent.
*Point de fusion (base) : 170-172°C*

| Microanalyse élémentaire : | | | |
|---|---|---|---|
| | **C** | **H** | **N** |
| % calculé | 49,20 | 4,72 | 12,30 |
| % Trouvé | 49,17 | 4,70 | 12,27 |

### EXEMPLE 5 : 2-(4,5-Dihydro-1H-2-imidazolyl)-2,4-diméthyl-3,4-dihydro-2H-1,4-benzoxazine, oxalate

### Stade a : 2,4-Diméthyl-2-méthoxycarbonyl-3,4-dihydro-2H-1,4-benzoxazine

A 11,3 mmol (2,5 g) de 2-carbéthoxy-4-méthyl-3,4-dihydro-2*H*-1,4-benzoxazine (décrite dans J. Heterocyclic. Chem., 1985, 22, 177) dans 10 ml d'éthanol et 10 ml de tétrahydrofurane, sont ajoutés 20 ml de KOH à 5 %. Le milieu réactionnel est agité à température ambiante pendant 1 heure. Le solvant est évaporé, et le résidu obtenu est repris dans un mélange acétate d'éthyle-eau, à pH acide, et extrait. La phase organique est séchée et concentrée. Le résidu est alors dissous dans 90 ml de tétrahydrofurane, et la température abaissée à - 50 °C, avant l'addition de 45,2 mmol (22,6 ml) de diisopropylamidure de lithium en solution 2 M dans le tétrahydrofurane. Le milieu réactionnel est agité 2 heures à - 50 °C puis 45,2 mmol (2,7 ml) d'iodométhane sont ajoutées, et la température est ramenée à 25 °C en 2 heures. Après hydrolyse, le milieu est acidifié jusqu'à pH = 4 et extrait à l'acétate d'éthyle. La phase organique est séchée et concentrée. Le résidu est alors dissous dans 150 ml de méthanol en présence d'acide paratoluène sulfonique. La réaction est chauffée à reflux pendant 18 heures. Le solvant est évaporé, et le milieu est repris par un mélange acétate d'éthyle-eau, et extrait. La phase organique est séchée, concentrée, et purifiée par chromatographie sur gel de silice en utilisant un mélange acétate d'éthyle / éther de pétrole, 3/7 , comme éluant, pour conduire au composé attendu.

### Stade b : 2-(4,5-Dihydro-1H-2-imidazolyl)-2,4-diméthyl-3,4-dihydro-2H-1,4-benzoxazine, oxalate

Le produit attendu est obtenu selon le procédé décrit dans l'exemple 1, stade b, à partir du composé décrit au stade précédent.

| Microanalyse élémentaire : | | | |
|---|---|---|---|
| | **C** | **H** | **N** |
| % calculé | 56,07 | 5,96 | 13,08 |
| % Trouvé | 56,40 | 6,10 | 12,55 |

### EXEMPLE 6 : 2-(4,5-Dihydro-1H-2-imidazolyl)-4-méthyl-2-propyl-3,4-dihydro-2H-1,4-benzoxazine, oxalate

### Stade a : 2-Méthoxycarbonyl-4-méthyl-2-propyl-3,4-dihydro-2H-1,4-benzoxazine

Le produit attendu est obtenu selon le procédé décrit dans l'exemple 5, stade a, en remplaçant l'iodométhane par le 1-iodopropane.

### Stade b : 2-(4,5-Dihydro-1H-2-imidazolyl)-4-méthyl-2-propyl-3,4-dihydro-2H-1,4-benzoxazine, oxalate

Le produit attendu est obtenu selon le procédé décrit dans l'exemple 1, stade b, à partir du composé obtenu au stade précédent.

| Microanalyse élémentaire : | | | |
|---|---|---|---|
| | **C** | **H** | **N** |
| % calculé | 58,44 | 6,63 | 12,03 |
| % Trouvé | 58,67 | 6,79 | 12,01 |

### EXEMPLE 7 : 2-(4,5-Dihydro-1H-2-imidazolyl)-2,4-diméthyl-3,4-dihydro-2H-1,4-benzoxazin-3-one

### Stade a : 2,4-Diméthyl-2-éthoxycarbonyl-3,4-dihydro-2H-1,4-benzoxazin-3-one

5,5 mmol (1,2 g) de 2-éthoxycarbonyl-3,4-dihydro-*2H*-1,4-benzoxazin-3-one (décrite dans C.R. Acad. Sci. Paris Série C, 1969, 269, 154) dans 5 ml de diméthylformamide sont ajoutées à 0 °C, à une suspension de 11 mmol (0,44 g) d'hydrure de sodium à 60 % dans 5 ml de diméthylformamide. Le milieu réactionnel est agité à 0 °C pendant 30 minutes. Sont ensuite ajoutées 22,2 mmol (1,38 ml) d'iodométhane et la réaction est agitée à température ambiante pendant 1 heure. Après hydrolyse et extraction à l'acétate d'éthyle, la phase organique est séchée, concentrée, et purifiée par chromatographie sur gel de silice en utilisant comme éluant un mélange acétate d'éthyle / éther de pétrole, 3/7, pour conduire au composé attendu.

### Stade b : 2-(4,5-Dihydro-1H-2-imidazolyl)-2,4-diméthyl-3,4-dihydro-2H-1,4-benzoxazin-3-one

Le produit attendu est obtenu selon le procédé décrit dans l'exemple 1, stade b, à partir du composé obtenu au stade précédent, en s'arrêtant au stade de la base libre.
*Point de fusion : 152-154°C*

| Microanalyse élémentaire : | | | |
|---|---|---|---|
| | **C** | **H** | **N** |
| % calculé | 63,65 | 6,18 | 17,13 |
| % Trouvé | 63,55 | 6,20 | 17,51 |

### EXEMPLE 8 : 2-(4,5-Dihydro-1H-2-imidazolyl)-2,3,6,7-tétrahydro-5H [1,4] oxazino [2,3,4-i,j] quinoline, oxalate

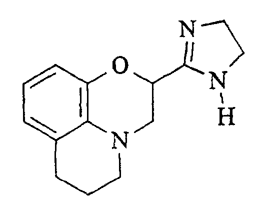

### Stade a : 4-[2-(Benzyloxycarbony)-éthyl]-2-éthoxycarbonyl-3,4-dihydro-2H-1,4-benzoxazine

A 22,6 mmol (5 g) de 2-éthoxycarbonyl-3,4-dihydro-*2H*-1,4-benzoxazine (décrite dans J. Heterocyclic. Chem., 1985, 22, 177) et 79 mmol (12,8 g) d'acrylate de benzyle, dans 17 ml de toluène sont ajoutées, à 0 °C, 2,3 mmol (0,9 ml) de triton B à 40 % dans l'eau. Le milieu réactionnel est porté à reflux pendant une nuit, le solvant est évaporé, et la réaction est chauffée à nouveau pendant 24 heures. Après refroidissement, hydrolyse et extraction à l'acétate d'éthyle, la phase organique est concentrée et purifiée par chromatographie sur gel de silice, en utilisant comme éluant un mélange acétate d'éthyle / éther de pétrole, 3/7, pour conduire au composé attendu.

### Stade b : 2-Ethoxycarbonyl-7-oxo-2,3,6,7-tétrahydro-5H[1,4] oxazino [2,3,4-i,j] quinoline

A 4 mmol (1,5 g ) du composé obtenu au stade précédent dans 20 ml d'éthanol, sont ajoutés 0,15 g de palladium sur charbon à 10 %. Le milieu réactionnel est agité sous atmosphère d'hydrogène pendant 4 heures, puis le catalyseur est filtré et le filtrat est concentré. L'anhydride trifluoroacétique (8 mmol) est ajouté, à 0 °C, au résidu obtenu, dissous dans 10 ml de dichlorométhane. Le milieu réactionnel est agité à température ambiante pendant 5 heures, puis hydrolysé. La réaction est ramenée à pH basique à l'aide de soude 5 N, et le milieu est extrait à l'acétate d'éthyle. La phase organique est concentrée et purifiée par chromatographie sur gel de silice en utilisant le dichlorométhane comme éluant, pour conduire au composé attendu.

### Stade c : 2-Ethoxycarbonyl-2,3,6,7-tétrahydro-5H[1,4]oxazino [2,3,4-i,j] quinoline

A 0,76 mmol (0,2 g) du composé décrit au stade précédent, dans 20 ml d'éthanol, sont ajoutés 50 mg de palladium sur charbon à 10 %. Le milieu réactionnel est agité sous atmosphère d'hydrogène pendant 48 heures. Le catalyseur est filtré, le filtrat est concentré, et purifié par chromatographie sur gel de silice en utilisant le dichlorométhane comme éluant, pour conduire au composé attendu.

### Stade d : 2-(4,5-Dihydro-1H-2-imidazolyl)-2,3,6,7-tétrahydro-5H[1,4]oxazino [2,3,4- i,j] quinoline, oxalate

Le produit attendu est obtenu selon le procédé décrit dans l'exemple 1, stade b, à partir du composé décrit au stade précédent.

| Microanalyse élémentaire : | | | |
|---|---|---|---|
| | **C** | **H** | **N** |
| % calculé | 57,65 | 5,74 | 12,61 |
| % Trouvé | 57,10 | 5,77 | 12,28 |

### EXEMPLE 9 : 2-(4,5-Dihydro-1H-2-imidazolyl)-8-méthyl-2,3,6,7-tétrahydro-5H [1,4] oxazino [2,3,4-i,j] quinoline, oxalate

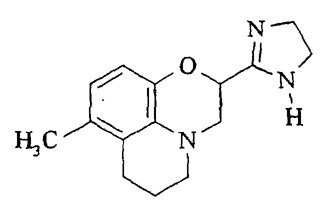

### Stade a : 4-[2-(Benzyloxycarbonyl)-éthyl]-2-éthoxycarbonyl-6-méthyl-3,4-dihydro-2H 1,4-benzoxazine

Le produit attendu est obtenu selon le procédé décrit dans l'exemple 8, stade a, à partir de la
2-éthoxycarbonyl-6-méthyl-3,4-dihydro-2H-1,4-benzoxazine.

### Stade b : 2-Ethoxycarbonoyl-8-méthyl-7-oxo-2,3,6,7-tétrahydro-5H[1,4]oxazino [2,3,4-i,j] quinoline

Le produit attendu est obtenu selon le procédé décrit dans l'exemple 8, stade b, à partir du composé décrit au stade précédent.

### Stade c : 2-(4,5-Dihydro-1H-2-imidazolyl)-8-méthyl-2,3,6,7-tétrahydro-5H [1,4] oxazino[2,3,4-i,j]quinoline, oxalate

Le produit attendu est obtenu selon le procédé décrit dans l'exemple 1, stade b, à partir du composé décrit au stade précédent.

| Microanalyse élémentaire : | | | |
|---|---|---|---|
| | C | H | N |
| % calculé | 58,78 | 6,09 | 12,10 |
| % Trouvé | 58,50 | 6,11 | 11,92 |

### EXEMPLE 10 : 2-(4,5-Dihydro-1H-2-imidazolyl)-7-hydroxy-2,3-dihydro-5H[1,4] oxazino[2,3,4-i,j] quinoline-5-one

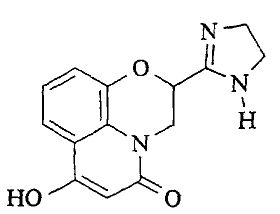

### Stade a : 4-[2-(Benzyloxycarbonyl)-acétyl]-2-éthoxycarbonyl-3,4-dihydro-2H-1,4-benzoxazine

A 17,1 mmol (3,53 g) de 2-éthoxycarbonyl-3,4-dihydro-2*H*-1,4-benzoxazine (décrit dans J. Heterocyclic. Chem., 1985, 22, 177) dans 35 ml de dichlorométhane, sont ajoutées 54,6 mmol (7,6 ml) de triéthylamine et 35,9 mmol (7,64 g) de 3-chloro-3-oxopropanoate de benzyle. Le milieu réactionnel est agité à température ambiante pendant une heure. Après hydrolyse et extraction au dichlorométhane, la phase organique est concentrée et purifiée par chromatographie sur gel de silice en utilisant un mélange acétate d'éthyle / éther de pétrole, 2/8) comme éluant, pour conduire au composé attendu.

### Stade b : 2-Ethoxycarbonyl-7-hydroxy-2,3-dihydro-5H[1,4] oxazino [2,3,4-i,j] quinolin-5-one

Le produit attendu est obtenu selon le procédé décrit dans l'exemple 8, stade b, à partir du composé décrit au stade précédent.

### Stade c : 2-(4,5-Dihydro-1H-2-imidazolyl)-7-hydroxy-2,3-dihydro-5H[1,4] oxazino[2,3,4-i,j] quinolin-5-one

Le produit attendu est obtenu selon le procédé décrit dans l'exemple 1, stade b, à partir du composé décrit au stade precedent. en s'arrêtant au stade de la base libre.

### EXEMPLE 11 : 2-(4,5-Dihydro-1H-2-imidazolyl)-7-hydroxy-8-méthyl-2,3-dihydro-5H[1,4]oxazino [2,3,4-i,j] quinolin-5-one

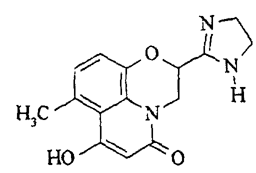

### Stade a : 4-[2-(Benzyloxycarbonyl)-acétyl]-2-éthoxycarbonyl-6-méthyl-3,4-dihydro-2H-1,4-benzoxazine

Le produit attendu est obtenu selon le procédé décrit dans l'exemple 10, stade a, en utilisant comme produit de départ la 2-éthoxycarbonyl-6-méthyl -3,4-dihydro-2*H*-1,4-benzoxazine.

### Stade b : 2-Ethoxycarbonyl-7-hydroxy-8-méthyl-2,3-dihydro-5H[1,4]oxazino [2,3,4-i,j] quinolin-5-one

Le produit attendu est obtenu selon le procédé décrit dans l'exemple 8, stade b, à partir du composé décrit au stade précédent.

### Stade c : 2-(4,5-Dihydro-1H-2-imidazolyl)-7-hydroxy-8-méthyl-2,3-dihydro-5H [1,4]oxazino[2,3,4-i,j]quinolin-5-one

Le produit attendu est obtenu selon le procédé décrit dans l'exemple 1, stade b, à partir du composé décrit au stade précédent, en s'arrêtant au stade de la base.

### EXEMPLE 12 : 2-(4,5-Dihydro-1H-2-imidazolyl)-7-méthoxy-2,3-dihydro-5H[1,4] oxazino[2,3,4-i,j] quinolin-5-one

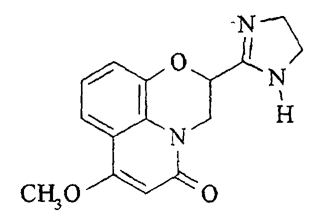

### Stade a : 2-Ethoxycarbonyl-7-méthoxy-2,3-dihydro-5H[1,4]oxazino[2,3,4-i,j] quinolin-5-one

A 3,6 mmol (1 g) du composé décrit dans l'exemple 10, stade b, dans 10 ml de diméthylformamide, sont ajoutées 7,3 mmol (1 g) de carbonate de potassium et 5,4 mmol (1 g) de tosylate de méthyle. Le milieu réactionnel est chauffé à 50 °C pendant 1 heure, puis hydrolysé et extrait à l'acétate d'éthyle. La phase organique est concentrée et purifiée par chromatographie sur gel de silice, en utilisant comme éluant le dichlorométhane, pour conduire au composé attendu.

### Stade b : 2-(4,5-Dihydro-1H-2-imidazolyl)-7-méthoxy-2,3-dihydro-5H[1,4] oxazino [2,3,4-i,j] quinolin-5-one

Le produit attendu est obtenu selon le procédé décrit dans l'exemple 1, stade b, à partir du composé décrit au stade précédent, en s'arrêtant au stade de la base libre.
*Point de fusion : 228-230°C*

| Microanalyse élémentaire : | | | |
|---|---|---|---|
| | **C** | **H** | **N** |
| % calculé | 63,15 | 5,30 | 14,73 |
| % Trouvé | 62,49 | 5,39 | 14,07 |

### EXEMPLE 13 : 2-(4,5-Dihydro-1H-2-imidazolyl)-7-méthoxy-8-méthyl-2,3-dihydro-5H[1,4]oxazino [2,3,4-i,j] quinolin-5-one

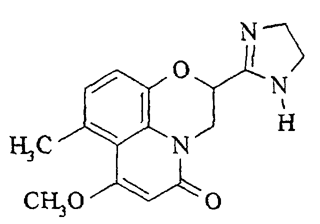

### Stade a : 2-Ethoxycarbonyl-7-méthoxy-8-méthyl-2,3-dihydro-5H[1,4] oxazino [2,3,4-i,j] quinolin-5-one

Le produit attendu est obtenu selon le procédé décrit dans l'exemple 12, stade a, à partir du composé décrit dans l'exemple 11, stade b.

### Stade b : 2-(4,5-Dihydro-1H-2-imidazolyl)-7-méthoxy-8-méthyl-2,3-dihydro-5H [1,4]oxazino [2,3,4-i,j] quinolin-5-one

Le produit attendu est obtenu selon le procédé décrit dans l'exemple 1, stade b, à partir du composé décrit au stade précédent, en s'arrêtant au stade de la base libre.
*Point de fusion : 222-224°C*

| Microanalyse élémentaire : | | | |
|---|---|---|---|
| | **C** | **H** | **N** |
| % calculé | 64,20 | 5,72 | 14,04 |
| % Trouvé | 64,02 | 5,87 | 13,36 |

### EXEMPLE 14 : 2-(4,5-Dihydro-1H-2-imidazolyl)-1,2,7,8,9,10-hexahydro-10a-aza-3-oxa-cyclohepta [d,e] naphtalène, oxalate

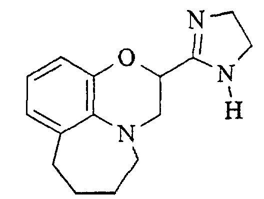

### Stade a : 4-[3-(Benzyloxycarbonyl)-propionyl]-2-éthoxycarbonyl-3,4-dihydro-2H-1,4- benzoxazine

Le produit attendu est obtenu selon le procédé décrit dans l'exemple 10, stade a, en remplaçant le 3-chloro-3-oxopropanoate de benzyle par le 4-chloro-4-oxobutanoate de benzyle.

### Stade b : 4-[3-(Benzyloxycarbonyl)-propyl]-2-éthoxycarbonyl-3,4-dihydro-2H-1,4-benzoxazine

A 10,8 mmol (4,3 g) du composé décrit au stade a, dans 20 ml de tétrahydrofurane, sont ajoutées 32,5 mmol (32,5 ml) de BH₃.THF en solution 1M dans le tétrahydrofurane. Le milieu réactionnel est chauffé à reflux pendant 4 heures. Après refroidissement, et évaporation du solvant, le résidu est repris dans un mélange eau-acétate d'éthyle, et extrait. La phase organique est séchée, concentrée et purifiée par chromatographie sur gel de silice, en utilisant comme éluant un mélange acétate d'éthyle / éther de pétrole, 3/7, pour conduire au composé attendu.

### Stade c : 2-Ethoxycarbonyl-7-oxo-1,2,7,8,9,10-hexahydro-3-oxa-10a-azacylcohepta[d,e]naphtalène

Le produit attendu est obtenu selon le procédé décrit dans l'exemple 8, stade b, à partir du composé décrit au stade précédent.

### Stade d : 2-Ethoxycarbonyl-1,2,7,8,9,10-hexahydro-3-oxa-10a-azacyclohepta[d,e]naphtalène

Le produit attendu est obtenu selon le procédé décrit dans l'exemple 8, stade c, à partir du composé décrit au stade précédent.

### Stade e : 2-(4,5-Dihydro-1H-2-imidazolyl)-1,2,7,8,9,10-hexahydro-3-oxa-10a-azacyclohepta [d,e] naphtalène, oxalate.

Le produit attendu est obtenu selon le procédé décrit dans l'exemple 1, stade b, à partir du composé décrit au stade précédent.

| Microanalyse élémentaire : | | | |
|---|---|---|---|
| | **C** | **H** | **N** |
| % calculé | 58,77 | 6,11 | 12,10 |
| % Trouvé | 58,67 | 6,12 | 11,87 |

### EXEMPLE 15 : 2-(4,5-Dihydro-1H-2-imidazolyl)-2,3,5,6-tétrahydro [1,4] oxazino [2,3,4-h,i] indole, oxalate

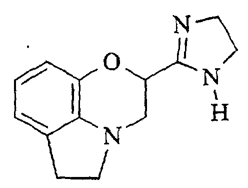

### Stade a : 7-Hydroxyindoline

Le produit attendu a été obtenu selon le procédé décrit dans J.Chem. Soc. (c), 1966, 344.

### Stade b : 2-Ethoxycarbonyl-2,3,5,6-tétrahydro [1,4] oxazino [2,3,4-h,i] indole

A une solution de 0,8 mmol (0,1 g) du composé décrit au stade précédent, dans 2 ml d'acétone, en présence de 2,1 mmol (0,3 g) de carbonate de potassium, sont ajoutées 0,85 mmol (0,12 ml) de 2,3-dibromopropionate d'éthyle. Le milieu réactionnel est chauffé à reflux pendant 18 heures. Après refroidissement et filtration, le solvant est évaporé. Le résidu est repris par un mélange eau-acétate d'éthyle, et extrait. La phase organique est séchée et purifiée par chromatographie sur gel de silice en utilisant comme éluant un mélange acétate d'éthyle / éther de pétrole, 1/9, pour conduire au composé attendu.

### Stade c : 2-(4,5-Dihydro-1H-2-imidazolyl)-2,3,5,6-tétrahydro[1,4]oxazino [2,3,4-h,i]indole, oxalate

Le produit attendu est obtenu selon le procédé décrit dans l'exemple 1, stade b, à partir du composé décrit au stade précédent.

| Microanalyse élémentaire : | | | |
|---|---|---|---|
| | **C** | **H** | **N** |
| % calculé | 56,42 | 5,37 | 13,16 |
| % Trouvé | 56,24 | 5,42 | 12,96 |

### EXEMPLE 16 : 2-(4,5-Dihydro-1H-2-imidazolyl)-2,3-dihydro[1,4]oxazino[2,3,4-h,i]indole, oxalate

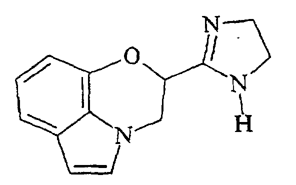

### Stade a : 2-Ethoxycarbonyl-2,3-dihydro[1,4]oxazino[2,3,4-h,i]indole

A une solution de 0,86 mmol (0,2 g) du composé décrit dans l'exemple 15, stade b, dans 6 ml de toluène, sont ajoutées 0,90 mmol (0,204 g) de DDQ à 0°C. Le milieu réactionnel est agité à cette température pendant 30 minutes avant d'être hydrolysé. Après neutralisation par une solution de soude à 5 % et extraction à l'acétate d'éthyle, les phases organiques regroupées sont séchées sur sulfate de magnésium, et concentrées. Une purification par chromatographie sur gel de silice en utilisant comme éluant un mélange acétate d'éthyle/éther de pétrole, 1/9, permet d'isoler le produit attendu.

### Stade b : 2-(4,5-Dihydro-1H-2-imidazolyl)-2,3-dihydro[1,4]oxazino[2,3,4-h,i]indole, oxalate

Le produit attendu est obtenu selon le procédé décrit dans l'exemple 1, stade b, à partir du composé décrit au stade précédent.
*Point de fusion : 190-192°C*

### EXEMPLE 17 : 2-(4,5-Dihydro-1H-2-imidazolyl)-4,7-diméthyl-3,4-dihydro-2H-1,4-benzoxazine, oxalate

### Stade a : 2-Ethoxycarbonyl-4,7-diméthyl-3,4-dihydro-2H-1,4-benzoxazine

Le produit attendu est obtenu selon le procédé décrit dans l'exemple 1, stade a, en remplaçant l'iodopropane par l'iodométhane et en utilisant comme produit de départ la 2-éthoxycarbonyl-7-méthyl-3,4-dihydro-2*H*-1,4-benzoxazine (préparée selon la méthode décrite dans J. Heterocyclic, Chem., 1985, *22*, 177, à partir du 2-amino-5-méthylphénol).

### Stade b : 2-(4,5-Dihydro-1H-2-imidazolyl)-4,7-diméthyl-3,4-dihydro-2H-1,4-benzoxazine, oxalate

Le produit attendu est obtenu selon le procédé décrit dans l'exemple 1, stade b, en utilisant comme produit de départ le composé décrit au stade précédent.
*Point de fusion (base) : 106-108°C*

### EXEMPLE 18 : 2-(4,5-Dihydro-1H-2-imidazolyl)-4,8-diméthyl-3,4-dihydro-2H-1,4-benzoxazine, oxalate

### Stade a : 2-Ethoxycarbonyl-4,8-diméthyl-3,4-dihydro-2H-1,4-benzoxazine

Le produit attendu est obtenu selon le procédé décrit dans l'exemple 1, stade a, en remplaçant l'iodopropane par l'iodométhane, et en utilisant comme produit de départ la 2-éthoxycarbonyl-8-méthyl-3,4-dihydro-2*H*-1,4-benzoxazine, préparée selon la méthode décrite dans J. Heterocyclic. Chem., 1985, *22*, 177, à partir du 2-amino-6-méthylphénol (décrit dans J. Org. Chem., 1996, 3289).

### Stade b : 2-(4,5-Dihydro-1H-2-imidazolyl)-4,8-diméthyl-3,4-dihydro-2H-1,4-benzoxazine, oxalate

Le produit attendu est obtenu selon le procédé décrit dans l'exemple 1, stade b, à partir du composé décrit au stade précédent.
*Point de fusion : 216-218°C*

### EXEMPLE 19 : 2-(4,5-Dihydro-1H-2-imidazolyl)-6-méthoxy-4-méthyl-3,4-dihydor-2H-1,4-benzoxazine, oxalate

### Stade a : 2-Ethoxycarbonyl-6-méthoxy-4-méthyl-3,4-dihydro-2H-1,4-benzoxazine

Le produit attendu est obtenu selon le procédé décrit dans l'exemple 1, stade a, en remplaçant l'iodopropane par l'iodométhane, et en utilisant comme produit de départ la 2-éthoxycarbonyl-6-méthoxy-2,3-dihydro-2*H*-1,4-benzoxazine, préparée selon la méthode décrite dans J. Heterocyclic. Chem., 1985, *22*, 177, à partir du 2-amino-4-méthoxyphénol (décrit dans J. Org. Chem., 1996, 3289).

### Stade b : 2-(4,5-Dihydro-1H-2-imidazolyl)-6-méthoxy-4-méthyl-3,4-dihydro-2H-1,4- benzoxazine, oxalate

Le produit attendu est obtenu selon le procédé décrit dans l'exemple 1, stade b, à partir du composé décrit au stade précédent.
*Point de fusion : 202-204°C*

### EXEMPLE 20 : 2-(4,5-Dihydro-1H-2-imidazolyl)-6-fluoro-4-méthyl-3,4-dihydro-2H-1,4- benzoxazine, oxalate

### Stade a : 2-Ethoxycarbonyl-6-fluoro-4-méthyl-3,4-dihydro-2H-1,4-benzoxazine

Le produit attendu est obtenu selon le procédé décrit dans l'exemple 1, stade a, en remplaçant l'iodopropane par l'iodométhane, et en utilisant comme produit de départ la 2-éthoxycarbonyl-6-fluoro-2,3-dihydro-2*H*-1,4-benzoxazine décrite dans la préparation A.

### Stade b : 2-(4,5-Dihydro-1H-2-imidazolyl)-6-fluoro-4-méthyl-3,4-dihydro-2H-1,4-benzoxazine, oxalate

Le produit attendu est obtenu selon le procédé décrit dans l'exemple 1, stade b, à partir du composé décrit au stade précédent.
*Point de fusion : 198-200°C*

### EXEMPLE 21 : 2-(4,5-Dihydro-1H-2-imidazolyl)-6-hydroxy-4-méthyl-3,4-dihydro-2H-1,4-benzoxazine, oxalate

A une solution de 0,57 mmol (140 mg) du composé décrit dans l'exemple 19, dans 5 ml de dichlorométhane à -10°C, sont ajoutées 1,14 mmol (0,11 ml) de tribromure de Bore. La réaction est chauffée à reflux pendant 1 heure. Le milieu réactionnel est hydrolysé et alcalinisé par une solution de soude à 10 %. Après extraction au dichlorométhane, les phases organiques sont regroupées, séchées sur sulfate de magnésium et concentrées. Une purification par chromatographie sur gel de silice en utilisant comme éluant un mélange dichlorométhane/triéthylamine, 100/1, permet d'obtenir le produit attendu. L'oxalate correspondant est obtenu par action d'une solution titrée d'acide oxalique dans l'éthanol.

### EXEMPLE 22 : 2-(4,5-Dihydro-1H-2-imidazolyl)-4-méthyl-6-trifluorométhyl-3,4-dihydro-2H-1,4-benzoxazine, oxalate

### Stade a : 2-Ethoxycarbonyl-4-méhtyl-6-trifluorométhyl-3,4-dihydro-2H-1,4-benzoxazine

Le produit attendu est obtenu selon le procédé décrit dans l'exemple 1, stade a, en remplaçant l'iodopropane par l'iodométhane et en utilisant comme produit de départ la 2-éthoxycarbonyl-6-trifluorométhyl-3,4-dihydro-2*H*-1,4-benzoxazine décrite dans la préparation B.

### Stade b : 2-(4,5-Dihydro-1H-2-imidazolyl)-4-méthyl-6-trifluorométhyl-3,4-dihydro-2H-1,4-benzoxazine, oxalate

Le produit attendu est obtenu selon le procédé décrit dans l'exemple 1, stade b, à partir du composé décrit au stade précédent.

### EXEMPLE 23 : 2-(4,5-Dihydro-1H-2-imidazolyl)-4-méthyl-8-trifluorométhyl-3,4-dihydro-2H-1,4-benzoxazine, oxalate

### Stade a : 2-Ethoxycarbonyl-4-méthyl-8-trifluorométhyl-3,4-dihydro-2H-1,4-benzoxazine

Le produit attendu est obtenu selon le procédé décrit dans l'exemple 1, stade a, en remplaçant l'iodopropane par l'iodométhane et en utilisant comme produit de départ 2-éthoxycarbonyl-8-trifluorométhyl-3,4-dihydro-2H-1,4-benzoxazine décrite dans la préparation C.

### Stade b : 2-(4,5-Dihydro-1H-2-imidazolyl)-4-méthyl-8-trifluorométhyl-3,4-dihydro-2H-1,4-benzoxazine, oxalate

Le produit attendu est obtenu selon le procédé décrit dans l'exemple 1, stade b, à partir du composé décrit au stade précédent.

### EXEMPLE 24 : 6-Acétyl-2-(4,5-dihydro-1H-2-imidazolyl)-4-méthyl-3,4-dihydro-2H-1,4-benzoxazine, oxalate

### Stade a : 6-Acétyl-2-éthoxycarbonyl-4-méthyl-3,4-dihydro-2H-1,4-benzoxazine

A une solution de 1,6 mmol (355 mg) de 2-éthoxycarbonyl-4-méthyl-3,4-dihydro-2*H*-1,4-benzoxazine (décrite dans J. Heterocyclic. chem., 1985, 22, 177) dans 18 ml de dichlorométhane à -10°C, sont ajoutées 1,6 mmol (0,1 ml) de chlorure d'acétyle et 4 mmol (545 mg) de chlorure d'aluminium. Le mélange réactionnel est agité à température ambiante pendant une heure avant d'être hydrolysé par une solution aqueuse refroidie d'acide chlorhydrique 2M. Le milieu est extrait au dichlorométhane et la phase organique est lavée par une solution saturée de NaHCO₃, séchée sur sulfate de magnésium, concentrée et purifiée par chromatographie sur gel de silice en utilisant comme éluant un mélange éther de pétrole/acétate d'éthyle, 7/3, pour conduire au produit attendu. Remarque : le composé 7-acétyl-2-éthoxycarbonyl-4-méthyl-3,4-dihydro-2*H*-benzoxazine est également isolé.

### Stade b : 6-Acétyl-2-(4,5-dihydro-1H-2-imidazolyl)-4-méthyl-3,4-dihydro-2H-1,4-benzoxazine, oxalate

Le produit attendu est obtenu selon le procédé décrit dans l'exemple 1, stade b, à partir du composé décrit au stade précédent.

### EXEMPLE 25 : 7-Acétyl-2-(4,5-dihydro-1H-2-imidazolyl)-4-méthyl-3,4-dihydro-2H-1,4- benzoxazine, oxalate

Le produit attendu est obtenu selon le procédé décrit dans l'exemple 1, stade b, en utilisant comme produit de départ la 7-acétyl-2-éthoxycarbonyl-4-méthyl-3,4-dihydro-2*H*-1,4-benzoxazine, isolée au stade a de l'exemple 24

### EXEMPLE 26 : 2-(4,5-Dihydro-1H-2-imidazolyl)-6-formyl-4-méhtyl-3,4-dihydro-2H-1,4-benzoxazine, oxalate

### Stade a : 2-Ethoxycarbonyl-6-formyl-4-méthyl-3,4-dihydro-2H-1,4-benzoxazine

A une solution de 4,3 mmol (950 mg) de 2-éthoxycarbonyl-4-méthyl-3,4-dihydro-2*H*-1,4-benzoxazine (décrite dans J. Heterocyclic. Chem., 1985, *22*, 177) dans 30 ml de dichlorométhane à -10°C sont ajoutées 6,5 mmol (0,6 ml) de dichlorométhylméthyléther et 6,5 mmol (0,7 ml) de tétrachlorure de titane. L'agitation est maintenue entre -10°C et 0°C pendant 2 heures. Le mélange réactionnel est hydrolysé à l'aide d'un mélange eau/glace, et extrait au dichlorométhane. La phase organique est séchée sur sulfate de magnésium, concentrée et purifiée par chromatographie sur gel de silice en utilisant comme éluant un mélange éther de pétrole/acétate d'éthyle, 7/3, pour conduire au produit du titre. Remarque : le composé 2-éthoxycarbonyl-7-formyl-4-méthyl-3,4-dihydro-2*H*-1,4-benzoxazine est également isolé.

### Stade b : 2-(4,5-Dihydro-1H-2-imidazolyl)-6-formyl-4-méhtyl-3,4-dihydro-2H-1,4-benzoxazine, oxalate

Le produit attendu est obtenu selon le procédé décrit dans l'exemple 1, stade b, à partir du composé décrit au stade précédent.

### EXEMPLE 27 : 2-(4,5-Dihydro-1H-2-imidazolyl)-7-formyl-4-méthyl-3,4-dihydro-2H-1,4-benzoxazine, oxalate

Le produit attendu est obtenu selon le procédé décrit dans l'exemple 1, stade b, en utilisant comme produit de départ la 2-éthoxycarbonyl-7-formyl-4-méthyl-3,4-dihydro-2H-1,4-benzoxazine isolée au stade a de l'exemple 26.

### EXEMPLE 28 : 2-(4,5-Dihydro-1H-2-imidazolyl)-7-méthoxy-4-méthyl-3,4-dihydro-2H-1,4-benzoxazine, oxalate

### Stade a : 2-Ethoxycarbonyl-7-méthoxy-4-méthyl-3,4-dihydro-2H-1,4-benzoxazine

Le produit attendu est obtenu selon le procédé décrit dans l'exemple 1, stade a, en remplaçant l'iodopropane par l'iodométhane et en utilisant comme produit de départ la 2-éthoxycarbonyl-7-méthoxy-3,4-dihydro-2*H*-1,4-benzoxazine décrite dans la préparation D.

### Stade b : 2-(4,5-Dihydro-1H-2-imidazolyl)-7-méthoxy-4-méthyl-3,4-dihydro-2H-1,4-benzoxazine, oxalate

Le produit attendu est obtenu selon le procédé décrit dans l'exemple 1, stade b, à partir du composé décrit au stade précédent.

### EXEMPLE 29 : 7-Chloro-2-(4,5-dihydro-1H-2-imidazolyl)-4-méthyl-3,4-dihydro-2H-1,4- benzoxazine, oxalate

### Stade a : 7-Chloro-2-éthoxycarbonyl-4-méthyl-3,4-dihydro-2H-1,4-benzoxazine

Le produit attendu est obtenu selon le procédé décrit dans l'exemple 1, stade a, en remplaçant l'iodopropane par l'iodométhane et en utilisant comme produit de départ la 7-chloro-2-éthoxycarbonyl-3,4-dihydro-2*H*-1,4-benzoxazine (obtenue selon le procédé décrit dans J. Heterocyclic. Chem., 1985, 22, 177, à partir de la 5-chloro-2-hydroxyaniline).

### Stade b : 7-Chloro-2-(4,5-dihydro-1H-2-imidazolyl)-4-méthyl-3,4-dihydro-2H-1,4- benzoxazine, oxalate

Le produit attendu est obtenu selon le procédé décrit dans l'exemple 1, stade b, à partir du composé décrit au stade précédent.

### EXEMPLE 30 : 2-(4,5-Dihydro-1H-2-imidazolyl)-4-méthyl-3,4,6,7,8,9-hexahydro-2H-naphto[2,3-b][1,4]oxazine, oxalate

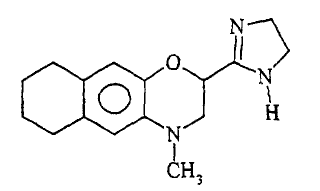

### Stade a : 2-Ethoxycarbonyl-4-méthyl-3,4,6,7,8,9-héxahydro-2H-naphto[2,3-b][1,4]oxazine

Le produit attendu est obtenu selon le procédé décrit dans l'exemple 1, stade a, en remplaçant l'iodopropane par l'iodométhane et en utilisant comme produit de départ la 2-éthoxycarbonyl-3,4,6,7,8,9-hexahydro-2*H*-naphto[2,3-b][1,4]oxazine, préparée selon le procédé décrit dans J. Heterocyclic. Chem., 1985, *22*, 177, à partuir du 3-amino-5,6,7,8-tétrahydro-2-naphtol (décrit dans Chem. Pharm. Bull., 1991, *39*, 2896).

### Stade b : 2-(4,5-Dihydro-1H-2-imidazolyl)-4-méthyl-3,4,6,7,8,9-hexahydro-2H-naphto[2,3-b][1,4]oxazine, oxalate

Le produit attendu est obtenu selon le procédé décrit dans l'exemple 1, stade b, à partir du composé décrit au stade précédent.
*Point de fusion (base) : 120-122°C*

### EXEMPLE 31 : 2-(4,5-Dihydro-1H-2-imidazolyl)-4-méthyl-3,4-dihydro-2H-naphto [1,2-b][1,4]oxazine, oxalate

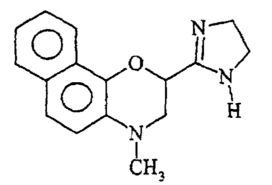

### Stade a : 2-Ethoxycarbonyl-4-méthyl-3,4-dihydro-2H-naphto[1,2-b][1,4]oxazine

Le produit attendu est obtenu selon le procédé décrit dans l'exemple 1, stade a, en remplaçant l'iodopropane par l'iodométhane et en utilisant comme produit de départ la 2-éthoxycarbonyl-3,4-dihydro-2*H*-naphto[1,2-b][1,4]oxazine, préparée selon le procédé décrit dans J. Heterocyclic. Chem., 1985, *22*, 177, à partir du 2-amino-1-naphtol.

### Stade b : 2-(4,5-Dihydro-1H-2-imidazolyl)-4-méthyl-3,4-dihydro-2H-naptho[1,2-b][1,4]oxazine, oxalate

Le produit attendu est obtenu selon le procédé décrit dans l'exemple 1, stade b, à partir du composé décrit au stade précédent.
*Point de fusion :* > *260°C*

### EXEMPLE 32 : 4-Cyclobutyl-2-<4.5-dihydro-1H-2-imidazolyl)-6-méthyl-3,4-dihydro-2H-1,4-benzoxazine, oxalate

Le produit attendu est obtenu selon le procédé décrit dans l'exemple 2, en remplaçant, au stade a, l'iodopropane par le chlorure de cyclobutyle.

### EXEMPLE 33 : 4-Cyclopropylméthyl-2-(4,5-dihydro-1H-2-imidazolyl)-6-méthyl-3,4-dihydro-2H-1,4-benzoxazine, oxalate

Le produit attendu est obtenu selon le procédé décrit dans l'exemple 2, en remplaçant, au stade a, l'iodopropane par le bromométhylcyclopropane.

### EXEMPLE 34 : 4-Benzyl-2-(4,5-dihydro-1H-2-imidazolyl)-6-méthyl-3,4-dihydro-2H-1,4- benzoxazine, oxalate

Le produit attendu est obtenu selon le procédé décrit dans l'exemple 2, en remplaçant, au stade a, l'iodopropane par le bromure de benzyle.

### EXEMPLE 35 : 2-Allyl-2-(4,5-dihydro-1H-2-imidazolyl)-4,6-diméthyl-3,4-dihydro-2H-1,4-benzoxazine, oxalate

Le produit attendu est obtenu selon le procédé décrit dans l'exemple 5 en remplaçant, au stade a, l'iodométhane par le bromure d'allyle.

### EXEMPLE 36 : 2-(4,5-Dihydro-1H-2-imidazolyl)-4,6-diméthyl-2-propyl-3,4-dihydro-2H-1,4-benzoxazine, oxalate

Le produit attendu est obtenu selon le procédé décrit dans l'exemple 5 en remplaçant, au stade a, l'iodométhane par l'iodopropane.

### EXEMPLE 37 : 2-Bromo-2-(4,5-dihydro-1H-2-imidazolyl)-4,6-diméthyl-3,4-dihydro-2H-1,4-benzoxazine, oxalate

### Stade a : 2-Bromo-2-éthoxycarbonyl-4,6-diméthyl-3,4-dihydro-2H-1,4-benzoxazine

A une solution de 3,1 mmol (730 mg) du composé décrit au stade a de l'exemple 2 dans 15 ml de tétrachlorure de carbone sont ajoutées 3,1 mmol (570 mg) de N-bromosuccinimide, et 50 mg de peroxyde de benzoyle. Le milieu réactionnel est chauffé à reflux pendant 5 heures. Après refroidissement, le précipité est filtré, et le filtrat est concentré et purifié par chromatographie sur gel de silice pour conduire au composé attendu.

### Stade b : 2-Bromo-2-(4, 5-dihydro-1H-2-imidazolyl)-4,6-diméthyl-3,4-dihydro-2H- 1,4-benzoxazine, oxalate

Le produit attendu est obtenu selon le procédé décrit dans l'exemple 1, stade b, à partir du composé décrit au stade précédent.

### EXEMPLE 38 : 2-(4,5-Dihydro-1H-2-imidazolyl)-2-méthoxy-4,6-diméthyl-3,4-dihydro-2H-1,4-benzoxazine, oxalate

### Stade a : 2-Ethoxycarbonyl-2-méthoxy-4-méthyl-3,4-dihydro-2H-1,4-benzoxazine

A une solution de 2,1-mmol du composé décrit au stade a de l'exemple 37, dans 8 ml de méthanol à 0°C, sont ajoutées 2,5 mmol d'éthylènediamine. Le milieu réactionnel est agité 18 heures à température ambiante puis hydrolysé par une solution saturée de NaHCO₃. Après extraction au dichlorométhane, les phases organiques regroupées sont séchées sur sulfate de magnésium et concentrées. Le résidu obtenu est purifié par chromatographie sur gel de silice en utilisant le dichlorométhane comme éluant, pour conduire au composé attendu.

### Stade b : 2-(4,5-Dihydro-1H-2-imidazolyl)-2-méthoxy-4,6-diméthyl-3,4-dihydro-2H-1,4-benzoxazine, oxalate

Le produit attendu est obtenu selon le procédé décrit dans l'exemple 1, stade b, à partir du composé décrit au stade précédent.

### EXEMPLE 39 : 2-Benzyloxy-2-(4,5-dihydro-1H-2-imidazolyl)-4,6-diméthyl-3,4-dihydro-2H-1,4-benzoxazine, oxalate

Le produit attendu est obtenu selon le procédé décrit dans l'exemple 38 en remplaçant, au stade a, le méthanol par l'alcool benzylique.

### EXEMPLE 40 : 2-(4,5-Dihydro-1H-2-imidazolyl)-4,6-diméthyl-4H-1,4-benzoxazine, oxalate

### Stade a : 4,6-Diméthyl-2-éthoxycarbonyl-4H-1,4-benzoxazine

Le produit attendu est obtenu selon le procédé décrit dans Tetrahedron Lett., 1978, 1059, en utilisant comme produit de départ le composé décrit dans l'exemple 2, stade a.

### Stade b : 2-(4,5-Dihydro-1H-2-imidazolyl)-4,6-diméthyl-4H-1,4-benzoxazine, oxalate

Le produit attendu est obtenu selon le procédé décrit dans l'exemple 1, stade b, à partir du composé décrit au stade précédent.

Selon le procédé décrit dans l'exemple 40, en utilisant comme produit de départ les 3,4-dihydro-2*H*-1,4-benzoxazines décrites dans les exemples précédents, les composés des exemples 41 à 44 sont obtenus.

### EXEMPLE 41 : 6-Chloro-2-(4,5-dihydro-1H-2-imidazolyl)-4-méthyl-4H-1,4-benzoxazine, oxalate

### EXEMPLE 42 : 2-(4,5-Dihydro-1H-2-imidazolyl)-4,7-diméthyl-4H-1,4-benzoxazine, oxalate

### EXEMPLE 43 : 2-(4,5-Dihydro-1H-2-imidazolyl)-5,6-dihydro[1,4]oxazino[2,3,4-h,i]indole, oxalate

### EXEMPLE 44 : 2-(4,5-Dihydro-1H-2-imidazolyl)-4-méthyl-6-trifluorométhyl-4H-1,4-benzoxazine, oxalate

En utilisant des procédés identiques à ceux décrits dans les exemples précédents, à partir de substrats à structure 2-éthoxycarbonyl-3,4-dihydro-2*H*-1,4-benzothiazines (J. Heterocyclic. Chem., 1985, 177, *22* ; ibid., 1980, 17, 377), les composés des exemples 45 à 60 sont obtenus.

### EXEMPLE 45 : 2-(4,5-Dihydro-1H-2-imidazolyl)-4,6-diméthyl-3,4-dihydro-2H-1,4-benzothiazine, oxalate

### EXEMPLE 46 : 6-Chloro-2-(4,5-dihydro-1H-2-imidazolyl)-4-méthyl-3,4-dihydro-2H-1,4-benzothiazine, oxalate

### EXEMPLE 47 : 2-(4,5-Dihydro-1H-2-imidazolyl)-4-méthyl-6-trifluorométhyl-3,4-dihydro-2H-1,4-benzothiazine, oxalate

### EXEMPLE 48 : 2-(4,5-Dihydro-1H-2-imidazolyl)-6-méthoxy-4-méhtyl-3,4-dihydro-2H-1,4-benzothiazine, oxalate

### EXEMPLE 49 : 6-Acétyl-(4,5-Dihydro-1H-2-imidazolyl)-4-méthyl-3,4-dihydro-2H-1,4- benzothiazine, oxalate

### EXEMPLE 50 : 2-(4,5-Dihydro-1H-2-imidazolyl)-4,7-diméthyl-3,4-dihydro-2H-1,4-benzothiazine, oxalate

### EXEMPLE 51 : 7-Chloro-2-(4,5-dihydro-1H-2-imidazolyl)-4-méthyl-3,4-dihydro-2H-1,4- benzothiazine, oxalate

### EXEMPLE 52 : 2-(4,5-Dihydro-1H-2-imidazolyl)-4-méthyl-8-trifluorométhyl-3,4-dihydro-2H-1,4-benzothiazine, oxalate

### EXEMPLE 53 : 4-Cyclopropylméthyl-2-(4,5-dihydro-1H-2-imidazolyl)-6-méthyl-3,4-dihydro-2H-1,4-benzothiazine, oxalate

### EXEMPLE 54 : 4-Benzyl-2-(4,5-dihydro-1H-2-imidazolyl)-6-méthyl-3,4-dihydro-2H-1,4- benzothiazine, oxalate

### EXEMPLE 55 : 6-Chloro-2-(4,5-Dihydro-1H-2-imidazolyl)-2,4-diméthyl-3,4-dihydro-2H- 1,4-benzothiazine, oxalate

### EXEMPLE 56 : 2-(4,5-Dihydro-1H-2-imidazolyl)-4,6-diméthyl-2-propyl-3,4-dihydro-2H- 1,4-benzothiazine, oxalate

### EXEMPLE 57 : 2-(4,5-Dihydro-1H-2-imidazolyl)-2,3,5,6-tétrahydro[1,4]thiazino [2,3,4-h,i]indole, oxalate

### EXEMPLE 58 : 2-(4,5-Dihydro-1H-2-imidazolyl)-2,3,6,7-tétrahydro-5H[1,6]thiazino [2,3,4-i,j]quinoline, oxalate

### EXEMPLE 59 : 2-(4,5-Dihydro-1H-2-imidazolyl)-4,6-diméthyl-4H-1,4-benzothiazine, oxalate

### EXEMPLE 60 : 2-(4,5-Dihydro-1H-2-imidazolyl)-4,7-diméthyl-4H-1,4-benzothiazine, oxalate

En utilisant des procédés identiques à ceux décrits dans les exemples 1 à 44, à partir de substrats à structure 2-éthoxycarbonyl-1,2,3,4-tétrahydroquinoline (Khim. Geterotsikl. Soedin., 1988, (1), 77), les composés des exemples 61 à 66 sont obtenus.

### EXEMPLE 61 : 3-(4,5-Dihydro-1H-2-imidazolyl)-1-méthyl-1,2,3,4-tétrahydroquinoline, oxalate

### EXEMPLE 62 : 3-(4,5-Dihydro-1H-2-imidazolyl)-1-propyl-1,2,3,4-tétrahydroquinoline, oxalate

### EXEMPLE 63 : 1-Cyclopropylméthyl-3-(4,5-dihydro-1H-2-imidazolyl)-1,2,3,4-tétrahydroquinoline, oxalate

### EXEMPLE 64 : 3-(4,5-Dihydro-1H-2-imidazolyl)-1-méthyl-3-propyl-1,2,3,4-tétrahydro-quinoline, oxalate

### EXEMPLE 65 : 7-Chloro-3-(4,5-dihydro-1H-2-imidazolyl-1-méthyl-1,2,3,4-tétrahydro-quinoline, oxalate

### EXEMPLE 66 : 3-(4,5-Dihydro-1H-2-imidazolyl)-7-méthoxy-1-méthyl-1,2,3,4-tétrahydro-quinoline, oxalate

### EXEMPLE 67 : 2-(4,5-Dihydro-1H-2-imidazolylméthyl)-4,6-diméthyl-3,4-dihydro-2H-1,4-benzoxazine, oxalate

### Stade a : 4,6-Diméthyl-2-hydroxyméthyl-3,4-dihydro-2H-1,4-benzoxazine

A une suspension de 40 mmol d'hydrure double d'aluminium et de lithium dans 100 ml de tétrahydrofurane à 0°C, sont ajoutées goutte à goutte 20 mmol du composé décrit au stade a de l'exemple 2 dans 15 ml de tétrahydrofurane. La réaction est agitée à température ambiante pendant une heure. Après hydrolyse, la solution est filtrée sur célite et le filtrat concentré. Le résidu est repris dans l'eau et extrait au dichlorométhane. La phase organique est séchée sur sulfate de magnésium, concentrée et purifiée par chromatographie sur gel de silice en utilisant comme éluant un mélange dichlorométhane/méthanol, 98/2, pour conduire au produit attendu.

### Stade b : 4,6-Diméthyl-2-iodométhyl-3,4-dihydro-2H-1,4-benzoxazine

A une solution de 6,1 mmol du composé décrit au stade précédent, dans 20 ml de toluène et 10 ml d'acétonitrile, sont ajoutées 13,4 mmol de triphénylphosphine et 27,4 mmol d'imidazole. Le mélange est porté à reflux puis 12,8 mmol d'iode sont ajoutées. La réaction est agitée à reflux pendant 1 heure. Après refroidissement, le solvant est évaporé, et le résidu est hydrolysé, puis extrait au dichlorométhane. La phase organique est séchée sur sulfate de magnésium, concentrée et purifiée par chromatographie sur gel de silice en utilisant comme éluant un mélange éther de pétrole/dichlorométhane, 5/5, pour conduire au composé attendu.

### Stade c : (4,6-Diméthyl-3,4-dihydro-2H-1,4-benzoxazin-2-yl)acétonitrile

A une solution de 4 mmol du composé décrit au stade précédent dans 6 ml de DMSO, sont ajoutées 28 mmol de cyanure de potassium. La réaction est agitée à température ambiante pendant 36 heures. Le milieu est ensuite dilué au dichlorométhane, et lavé 10 fois à l'eau. La phase organique est séchée sur sulfate de magnésium, concentrée et purifiée par chromatographie sur gel de silice en utilisant comme éluant un mélange dichlorométhane/éther de pétrole, 5/5, pour conduire au composé attendu.

### Stade d : 2-(4,6-Diméthyl-3,4-dihydro-2H-1,4-benzoxazin-2-yl)acétate d'éthyle

Le composé décrit au stade précédent dissout dans une solution de soude à 10 % est chauffé à reflux pendant 50 minutes. Après refroidissement, le milieu est acidifié par une solution d'acide chlorhydrique 2N et extrait au dichlorométhane. La phase organique est séchée sur sulfate de magnésium et concentrée. Le résidu est dissous dans l'éthanol en présence d'une quantité catalytique d'acide paratoluène sulfonique. La réaction est chauffée à reflux pendant 18 heures. Après refroidissement, le solvant est évaporé, le résidu est repris par un mélange acétate d'éthyle-eau et extrait. La phase organique est séchée, concentrée et purifiée par chromatographie sur gel de silice en utilisant comme éluant un mélange éther de pétrole/acétate d'éthyle, 7/8, pour conduire au produit attendu.

### Stade e : 2-(4,5-Dihydro-1H-2-imidazolylméthyl)-4,6-diméthyl-3,4-dihydro-2H-1,4- benzoxazine, oxalate

Le produit attendu est obtenu selon le procédé décrit dans l'exemple 1, stade b, à partir du composé décrit au stade précédent.

Les composés des exemples 68 à 75 sont obtenus selon un procédé identique à celui décrit dans l'exemple 67 en utilisant comme substrat les dérivés appropriés décrits dans les exemples 1 à 66.

### EXEMPLE 68 : 2-(4,5-Dihydro-lH-2-imidazolylméthyl)-2,4-diméthyl-3,4-dihydro-2H-1,4-benzoxazine, oxalate

### EXEMPLE 69 : 2-(4,5-Dihydro-1H-2-imidazolylméthyl)-4-méthyl-6-trifluorométhyl-3,4- dihydro-2H-1,4-benzoxazine, oxalate

### EXEMPLE 70 : 4-Cyclopropylméthyl-2-(4,5-dihydro-1H-2-imidazolylméthyl)-6-méthyl-3,4-dihydro-2H-1,4-benzoxazine, oxalate

### EXEMPLE 71 : 2-(4,5-Dihydro-1H-2-imidazolylméthyl)-4,6-diméthyl-3,4-dihydro-2H-1,4-benzothiazine, oxalate

### EXEMPLE 72 : 2-(4,5-Dihydro-1H-2-imidazolytméthyl)-6-méthoxy-4-méthyl-3,4-dihydro-2H-1,4-benzothiazine, oxalate

### EXEMPLE 73 : 2-(4,5-Dihydro-1H-2-imidazolylméthyl)-4,6-diméthyl-2-propyl-3,4-dihydro-2H-1,4-benzothiazine, oxalate

### EXEMPLE 74 : 3-(4,5-Dihydro-1H-2-imidazolylméthyl)-1-méthyl-1,2,3,4-tétrahydroquinoline, oxalate

### EXEMPLE 75 : 3-(4,5-Dihydro-1H-2-imidazolylméthyl)-7-méthoxy-1-méthyl-1,2,3,4-tétrahydroquinoline, oxalate

### ETUDE PHARMACOLOGIQUE DES COMPOSES DE L'INVENTION

### EXEMPLE A : Mesure de l'affinité in vitro aux récepteurs I₁, I₂

Le profil de liaison aux récepteurs imidazolines I₁ et I₂ a été déterminé pour les composés de l'invention en évaluant la capacité de ces dérivés à déplacer les radioligands spécifiques des récepteurs concernés.
Le tableau suivant indique le radioligand utilisé pour marquer chaque récepteur, le produit et la concentration retenue pour déterminer la fraction non spécifique et le tissu choisi.

| ***Récepteur ou site*** | ***Radioligand*** | ***Non spécifique*** | ***Tissus*** |
|---|---|---|---|
| I₁ | [³H] clonidine + 10µM noradrénaline | 10⁻⁵ M clonidine | Noyau réticulé latéral de boeuf |
| I₂ | [³H] idazoxan + 10µm noradrénaline | 10⁻⁵ M idazoxan | cortex rénal de lapin |

### Résultats .

Les résultats obtenus révèlent pour les composés de l'invention une très haute affinité pour les récepteurs I₁ et I₂, variant de quelques nanomoles à quelques dizaines de nanomoles.
A titre d'exemple, le composé de l'exemple 2 présente des Ki de 2,2,10⁻⁸M et 2,0.10-⁹M pour les récepteurs I₁ et I₂ respectivement.

### EXEMPLE B : Mise en évidence de l'activité antihypertensive chez le rat anesthésié

Les expériences ont été réalisées chez le rat SHR mâle âgé de 18 semaines. Les rats sont anesthésiés au pentobarbital (50 mg/kg i.p.). Un cathéter est introduit dans l'artère carotide gauche, de façon à enregistrer la pression artérielle systolique et diastolique, ainsi que la fréquence cardiaque. Seuls sont inclus dans l'étude les animaux dont la pression artérielle est supérieure ou égale à 170 mmHg.
On laisse les paramètres à mesurer se stabiliser pendant une période d'au moins 30 minutes. Le composé à étudier, ou le véhicule, est administré par voie intrapéritonéale, à une dose de 25 mg/kg (le produit est mis en suspension dans de l'eau distillée contenant 0,5 % de carboxyméthylcellulose, le volume d'injection étant de 0,25 ml / 100 g), les animaux contrôle recevant uniquement le véhicule.
Les mesures effectuées sont exprimées en mmHg pour la pression artérielle et en bpm pour la fréquence cardiaque et sont comparées aux valeurs basales.
Un composé est considéré comme actif si l'effet enregistré sur la pression artérielle ou la fréquence cardiaque est supérieure à 25 mmHg ou 50 bpm, respectivement.

### Résultats

Il apparaît que les composés de l'invention sont capables de diminuer de façon significative la pression artérielle ainsi que la fréquence cardiaque.

### EXEMPLE C : Mise en évidence de l'activité hypotensive chez le lapin normotendu anesthésié

Les expériences sont réalisées chez le lapin normotendu mâle. Les lapins sont anesthésiés au pentobarbital (40 mg/kg; i.v.), artificiellement ventilés puis curarisés. La pression artérielle et la fréquence cardiaque sont mesurées à l'aide d'un cathéter introduit dans l'artère fémorale droite.
Avant chaque expérience, un volume égal de véhicule est administré, et on laisse les paramètres à mesurer se stabiliser. Le composé à tester ou le véhicule sont ensuite administrés, soit par voie intracisternale, soit par voie intraveineuse, à des doses croissantes et cumulatives comprises entre 1 et 300 µg/kg.
Les mesures de la pression artérielle systolique et diastolique instantanée, ainsi que de la fréquence cardiaque sont effectuées entre 5 et 10 minutes après les injections.

### Résultats : Les résultats sont exprimés en pourcentage de variation par rapport aux valeurs basales.

Il apparaît que les composés de l'invention diminuent de façon significatives la pression artérielle et la fréquence cardiaque, avec un effet dose-dépendant.
A titre d'exemple, le composé de l'exemple 2 induit une baisse comprise entre 19 et 30 % pour la pression artérielle et comprise entre 9 et 22 % pour la fréquence cardiaque.

### EXEMPLE D : Mise en évidence de l'activité antihypertensive chez le rat conscient

Les expériences sont réalisées chez le rat SHR. Les animaux sont anesthésiés. Un cathéter heparinisé est introduit dans l'artère carotide gauche et relié à un appareil enregistreur de façon à mesurer la pression artérielle systolique et diastolique, ainsi que la fréquence cardiaque. Les animaux sont replacés dans leur cage et les expériences sont réalisées après 24 heures.
Les paramètres à mesurer se stabilisent pendant une période d'au moins 30 minutes, durant laquelle un maximum de 1,5 ml d'une solution saline est injectée de manière à optimiser les mesures. Seuls sont inclus dans l'étude les animaux dont la pression artérielle est supérieure ou égale à 170 mmHg.
Les produits à tester ou le véhicule sont administrés par voie orale et les mesures sont effectuées avant et toutes les dix minutes après le traitement, pendant 4 heures, puis 8 et 24 heures après le traitement.

### Résultats :

Il apparaît que les composés de l'invention sont capables de diminuer de façon significative la pression artérielle ainsi que la fréquence cardiaque.

A titre d'exemple, le composé de l'exemple 2 administré à une dose de 15 mg/kg permet d'observer une normalisation de la pression artérielle.

### EXEMPLE E : Toxicité aiguë

La toxicité aiguë a été appréciée après administration orale à des lots de 8 souris (26 ± 2 grammes) d'une dose de 650 mg.kg⁻¹. Les animaux ont été observés à intervalles réguliers au cours de la première journée et quotidiennement pendant les deux semaines suivant le traitement.
Il apparaît que la plupart des composés de l'invention sont totalement atoxiques. La plupart d'entre eux n'entraîne aucun décès après administration à une dose de 650 mg.kg⁻¹ et on ne constate généralement pas de troubles après administration de cette dose.

### EXEMPLE F : Composition pharmaceutique

| Formule de préparation pour 1000 comprimés dosés à 10 mg. | |
|---|---|
| Composé de l'exemple 2 | 10 g |
| Hydroxypropylcellulose | 2 g |
| Amidon de blé | 10 g |
| Amidon de mais | 10 g |
| Lactose | 100 g |
| Stéarate de magnésium | 3 g |
| Talc | 3 g |

## Revendications

1. Composés de formule (I) : 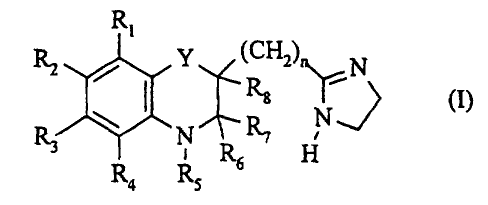 dans laquelle :
• n est égal à 0 ou 1,
• Y représente un atome d'oxygène, de soufre, ou un groupement CH₂,
• R₁, R₂, R₃, R₄, identiques ou différents, représentent un atome d'hydrogène, d'halogène, un groupement alkyle (C₁-C₆) linéaire ou ramifié, cycloalkyle (C₃-C₇), alkoxy (C₁-C₆) linéaire ou ramifié, hydroxy, hydroxyalkyle (C₁-C₆) linéaire ou ramifié, trihalogénoalkyle (C₁-C₆) linéaire ou ramifié, alkyle (C₁-C₆) carbonyle linéaire ou ramifié, formyle, cyano, carboxy, alkoxy (C₁-C₆) carbonyle linéaire ou ramifié, nitro, amino éventuellement substitué,
ou bien, (R₁-R₂) ou (R₂-R₃) ou (R₃-R₄) forment avec les carbones auxquels ils sont liés un cycle à 5 ou 6 chaînons saturé ou insaturé éventuellement substitué,
• R₅ représente un atome d'hydrogène, un groupement cycloalkyle (C₃-C₇), ou un groupement alkyle (C₁-C₆) linéaire ou ramifié éventuellement substitué par un groupement choisi parmi cycloalkyle (C₃-C₇) et phényle éventuellement substitué,
ou forme avec R₄ un cycle à 5, 6 ou 7 chaînons saturé ou insaturé, éventuellement substitué par un ou plusieurs groupements, identiques ou différents, choisis parmi alkyle (C₁-C₆) linéaire ou ramifié, alkoxy (C₁-C₆) linéaire ou ramifié, hydroxy, oxo et amino éventuellement substitué,
• R₆ et R₇, représentent chacun un atome d'hydrogène ou forment ensemble un groupement oxo,
• R₈ représente un atome d'halogène, d'hydrogène ou un groupement alkyle (C₁-C₆) linéaire ou ramifié, alkényle (C₂-C₆) linéaire ou ramifié, hydroxy, alkoxy (C₁-C₆) linéaire ou ramifié (éventuellement substitué par un groupement amino, pyrolyle, pipéridinyle), cycloalkyloxy (C₃-C₇), phényloxy éventuellement substitué, benzyloxy éventuellement substitué,
ou forme avec R₇ une liaison,
étant entendu que :
- lorsque n est égal à 0, Y représente un atome d'oxygène et R₁, R₂, R₃, R₄, R₆, R₇, R₈ représentent chacun un atome d'hydrogène, alors R₅ est différent d'un atome d'hydrogène, d'un groupement méthyle, éthyle ou benzyle,
- lorsque n est égal à 0, Y représente un atome de soufre et R₁, R₂, R₃, R₄, R₆, R₇, R₈ représentent chacun un atome d'hydrogène, alors R₅ est différent d'un atome d'hydrogène,
- par groupement amino éventuellement substitué, on entend substitué par un ou deux groupements, identiques ou différents, choisis paru alkyle (C₁-C₆) linéaire ou ramifié, phényle éventuellement substitué, benzyle éventuellement substitué,
- les termes éventuellement substitué, affectés aux groupements phényloxy, benzyloxy, phényle et benzyle ainsi qu'à l'expression "cycle à 5 ou 6 chaînons", signifient que ces groupements peuvent être substitués par un ou plusieurs substituants, identiques ou différents, choisis parmi halogène, alkyle (C₁-C₆) linéaire ou ramifié, alkoxy (C₁-C₆) linéaire ou ramifié, hydroxy et trihalogénoalkyle (C₁-C₆) linéaire ou ramifié,
leurs N-oxydes, leurs énantiomères, diastéréoisomères, ainsi que leurs sels d'addition à un acide ou à une base pharmaceutiquement acceptable.

2. Composés de formule (I) selon la revendication 1 tels que n est égal à 0, leurs N-oxydes, leurs énantiomères, diastéréoisomères, ainsi que leurs sels d'addition à un acide ou à une base pharmaceutiquement acceptable.

3. Composés de formule (I) selon la revendication 1 tels que Y représente un atome d'oxygène, leurs N-oxydes, leurs énantioméres, diastéréoisomères, ainsi que leurs sels d'addition à un acide ou à une base pharmaceutiquement acceptable.

4. Composés de formule (I) selon la revendication 1 tels que Y représente un atome de soufre, leurs N-oxydes, leurs énantiomères, diastéréoisomères, ainsi que leurs sels d'addition à un acide ou à une base pharmaceutiquement acceptable.

5. Composés de formule (I) selon la revendication 1 tels que R₆, R₇ et R₈ représentent chacun un atome d'hydrogène, leurs N-oxydes, leurs énantiomères, diastéréoisomères, ainsi que leurs sels d'addition à un acide pharmaceutiquement acceptable.

6. Composés de formule (I) selon la revendication 1 tels que R₅ représente un groupement alkyle (C₁-C₆) linéaire ou ramifié, leurs N-oxydes, leurs énantiomères, diastéréoisomères, ainsi que leurs sels d'addition à un acide pharmaceutiquement acceptable.

7. Composés de formule (I) selon la revendication 1 tels que trois des groupements R₁, R₂, R₃ et R₄ sont identiques et représentent chacun un atome d'hydrogène, le groupement restant étant tel que défini dans la formule (I), leurs N-oxydes, leurs énantiomères, diastéréoisomères, ainsi que leurs sels d'addition à un acide pharmaceutiquement acceptable.

8. Composés de formule (I) selon la revendication 1 tels que (R₁-R₂) ou (R₂-R₃) ou (R₃-R₄) forment avec les carbones auxquels ils sont liés un cycle à 5 ou 6 chaînons saturé ou insaturé éventuellement substitué, leurs N-oxydes, leurs énantiomères, diastéréoisomères, ainsi que leurs sels d'addition à un acide pharmaceutiquement acceptable.

9. Composés de formule (I) selon la revendication 1 tels que R₄ et R₅ forment ensemble un cycle à 5, 6 ou 7 chaînons, saturé ou insaturé, éventuellement substitué par un ou plusieurs groupements, identiques ou différents, choisis parmi alkyle (C₁-C₆) linéaire ou ramifié, alkoxy (C₁-C₆) linéaire ou ramifié, hydroxy, oxo et amino éventuellement substitué, leurs N-oxydes, leurs énantiomères, diastéréoisomères, ainsi que leurs sels d'addition à un acide ou à une base pharmaceutiquement acceptable.

10. Composés de formule (I) selon la revendication 1 tels que Y représente un atome d'oxygène, n vaut 0, R₆, R₇ et R₈ représentent chacun un atome d'hydrogène, R₁, R₂, R₃, R₄, identiques ou différents, représentent un atome d'hydrogène, d'halogène, un groupement alkyle (C₁-C₆) linéaire ou ramifié, cycloalkyle (C₃-C₇), alkoxy (C₁-C₆) linéaire ou ramifié, hydroxy, hydroxyalkyle (C₁-C₆) linéaire ou ramifié, trihalogénoalkyle (C₁-C₆) linéaire ou ramifié, alkyle (C₁-C₆) carbonyle linéaire ou ramifié, formyle, cyano, carboxy, alkoxy (C₁-C₆) carbonyle linéaire ou ramifié, nitro, amino éventuellement substitué, ou bien (R₁-R₂) ou (R₂-R₃) ou (R₃-R₄) forment avec les carbones auxquels ils sont liés un cycle à 5 ou 6 chaînons saturé ou insaturé éventuellement substitué, tandis que R₅ représente un groupement alkyle (C₁-C₆) linéaire ou ramifié, ou forme avec R₄ un cycle à 5, 6 ou 7 chaînons, saturé ou insaturé, leurs N-oxydes, leurs énantiomères, diastéréoisomères, ainsi que leurs sels d'addition à un acide ou à une base pharmaceutiquement acceptable.

11. Composés de formule (I) selon la revendication 1 qui sont les :
- 2-(4,5-dihydro-1*H*-2-imidazolyl)-4,6-diméthyl-3,4-dihydro-2*H*-1,4-benzoxazine,
- 2-(4,5-dihydro-1*H*-2-imidazolyl)-4,7-diméthyl-3,4-dihydro-2*H*-1,4-benzoxazine,
- 2-(4,5-dihydro-1*H*-2-imidazolyl)-4,8-diméthyl-3,4-dihydro-2H-1,4-benzoxazine,
leurs N-oxydes, leurs énantiomères, diastéréoisomères, ainsi que leurs sels d'addition à un acide pharmaceutiquement acceptable.

12. Composé de formule (I) selon la revendication 1 qui est le 6-chloro-2-(4,5-dihydro-1*H*-2-imidazolyl)-4-méthyl-3,4-dihydro-2*H*-1,4-benzoxazine, ses N-oxydes, ses énantiomères, diastéréoisomères, ainsi que ses sels d'addition à un acide pharmaceutiquement acceptable.

13. Composés de formule (I) selon la revendication 1 qui sont les :
- 2-(4,5-dihydro-1*H*-2-imidazolyl)-2,3,5,6-tétrahydro[1,4]oxazino[2,3,4-*h*,*i*]indole,
- 2-(4,5-dihydro-1*H*-2-imidazolyl)-2,3-dihydro[1,4]oxazino[2,3,4-*h*,*i*]indole,
leurs N-oxydes, leurs énantiomères, diastéréoisomères, ainsi que leurs sels d'addition à un acide pharmaceutiquement acceptable.

14. Procédé de préparation des composés de formule (I) caractérisé :
• lorsque dans le composé de formule (I) que l'on souhaite obtenir, R₄ et R₅ ne forment pas ensemble un cycle, en ce que l'on utilise comme matière première un composé de formule (II/a): 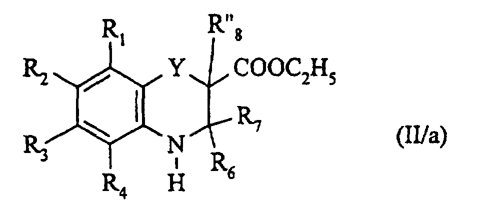 dans laquelle R"₈ représente un atome d'hydrogène, ou forme avec R₇ une liaison et Y, R₁, R₂, R₃, R₄, R₆ et R₇ sont tels que définis dans la formule (I),
que l'on traite,
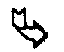 soit par un dérivé de formule (III) :
R'₅-X (III)
dans laquelle X représente un atome d'halogène et R'₅ représente un groupement cycloalkyle (C₃-C₇), ou un groupement alkyle (C₁-C₆) linéaire ou ramifié éventuellement substitué par un groupement cycloalkyle (C₃-C₇), phényle éventuellement substitué,
pour conduire à un composé de formule (II/b): 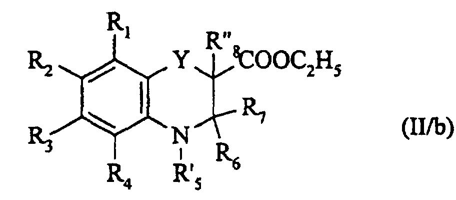 dans laquelle Y, R₁, R₂, R₃, R₄, R'₅, R₆, R₇ et R"₈ sont tels que définis précédemment,
qui peut être soumis, lorsque R"₈ représente un atome d'hydrogène, à l'action d'un agent d'halogénation, ou bien à l'action d'une base forte suivie d'un traitement par un dérivé de formule (IV) :
R'₈-X (IV)
dans laquelle X représente un atome d'halogène et R'₈, différent d'un atome d'hydrogène, a la même signification que R₈ dans la formule (I),
pour conduire à un composé de formule (II/c) : 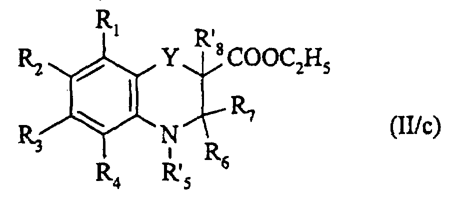 dans laquelle Y, R₁, R₂, R₃, R₄, R'₅, R₆ et R₇ et R'₈ sont tels que définis précédemment,
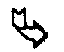 soit, lorsque dans le composé de formule (II/a) utilisé, R"₈ représente un atome d'hydrogène, par un dérivé de formule (IV) telle que définie précédemment, après un traitement par une base forte,
pour conduire à un composé de formule (II/d): 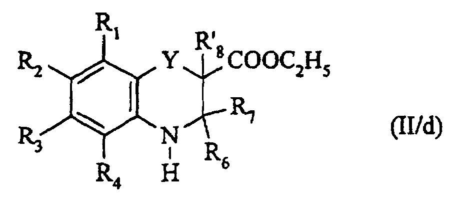 dans laquelle Y, R₁, R₂, R₃, R₄, R₆ R₇ et R'₈ sont tels que définis précédemment,
composés (II/a), (II/b), (II/c) et (II/d), formant la totalité des composés de formule (II) : 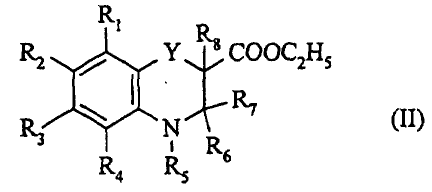 dans laquelle Y, R₁, R₂, R₃, R₄, R₅, R₆, R₇ et R₈ sont tels que définis précédemment,
que l'on traite, éventuellement après homologation d'un carbone de la chaine portant la fonction ester, par l'éthylènediamine pour conduire à un composé de formule (I/a) 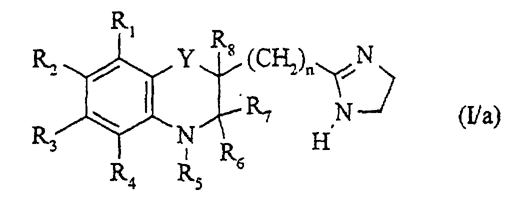 cas particulier des composés de formule I, pour lequel, Y, R₁, R₂, R₃, R₄, R₅, R₆, R₇, R₈ et n ont la même signification que dans la formule (I), étant entendu que R₄ et R₅ ne forment pas ensemble un cycle,
ou bien,
• lorsque dans le composé de formule (I) que l'on souhaite obtenir, R₄ et R₅ forment ensemble un cycle, en ce que l'on utilise comme matière première un composé de formule (II/e) : 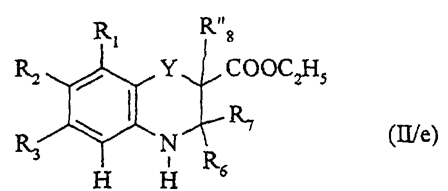 dans laquelle R₁, R₂, R₃, R₆, R₇ et R"₈ sont tels que définis précédemment,
que l'on traite par un dérivé de formule (V) ou (VI) : 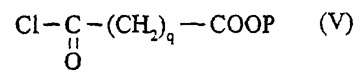 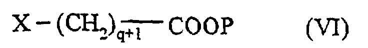 dans lesquelles X représente un atome d'halogène ou un groupe partant, P un groupement masquant la fonction acide, et q un entier égal à 0, 1 ou 2,
pour conduire à un composé de formule (VII) : 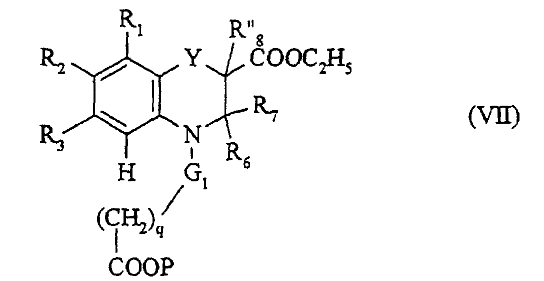 dans laquelle G₁ représente un groupement CH₂ ou CO, et q, P, Y, R₁, R₂, R₃, R₆, R₇ et R"₈ ont la même signification que précédemment,
qui, après libération de la fonction acide et cyclisation, conduit au composé de formule (VIII/a) : 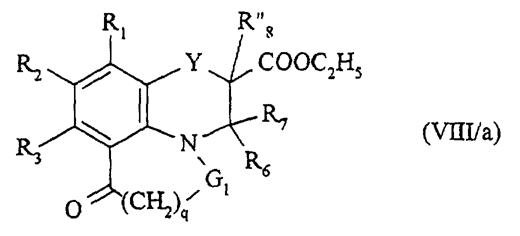 dans laquelle Y, R₁, R₂, R₃, R₆, R₇, R"₈, G₁ et q ont la même signification que précédemment,
composé (VIII/a),
- qui peut être transformé, après traitement par un halogénure d'alkyle, en un composé de formule (VIII/b) : 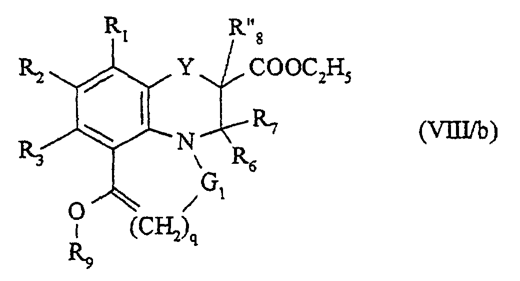 dans laquelle R₉ représente un groupement alkyle (C₁-C₆) linéaire ou ramifié, et Y, R₁, R₂, R₃, R₅, R₇, R"₈, G₁, et q ont la même signification que précédemment,
- ou peut être réduit pour conduire à un composé de formule (VIII(c) : 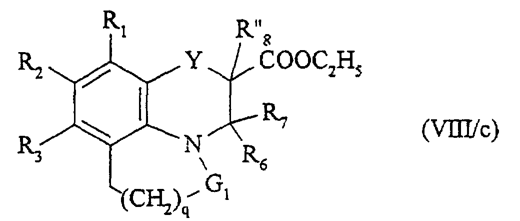 dans laquelle Y, R₁, R₂, R₃, R₆, R₇, R"₈, G₁ et q sont tels que définis précédemment,
chacun des composés (VIII/a), (VIII/b) et (VIII/c) pouvant être traité, lorsque cela est compatible avec les substituants présents sur la molécule, par une base forte, puis par un dérivé de formule (IV) telle que définie précédemment, pour conduire au composé de formule (VIII/d) : 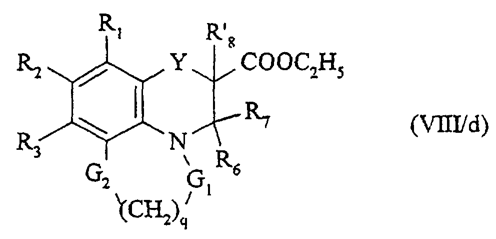 dans laquelle G₂ représente un groupement CH₂, CO, ou forme avec le carbone du méthylène adjacent un groupement 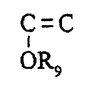 et Y, R₁, R₂, R₃, R₆, R₇, R'₈, R₉, G₁ et q sont tels que définis précédemment,
composés (VIII/a), (VIII/b), (VIII/c), et (VIII/d) formant la totalité des composés de formule (VIII) : 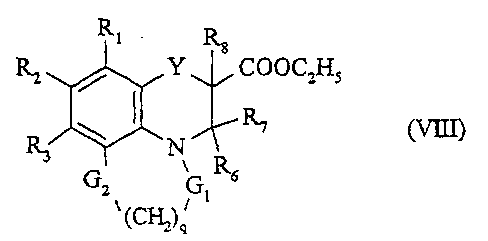 dans laquelle R₁, R₂, R₃, R₆, R₇, R₈, G₁, G₂ et q sont tels que définis précédemment,
et qui sont traités, éventuellement après homologation d'un carbone de la chaîne portant la fonction ester, par l'éthylènediamine pour conduire à un composé de formule (I/b) : 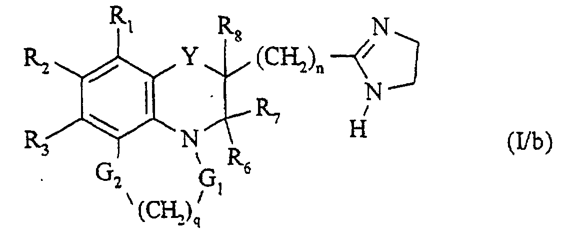 cas particulier des composés de formule (I), pour lequel Y, R₁, R₂, R₃, R₆, R₇, R₈, G₁, G₂, n et q sont tels que définis précédemment,
composés (I/a) et (I/b) :
- que l'on purifie éventuellement selon une technique classique de purification,
- dont on sépare éventuellement les isomères selon une technique classique de séparation,
- et que l'on transforme, le cas échéant, en leurs N-oxydes ou en leurs sels d'addition à un acide ou à une base pharmaceutiquement acceptable.

15. Procédé de préparation des composés de formule (I) caractérisé en ce que l'on utilise comme matière première un composé de formule (IX) : 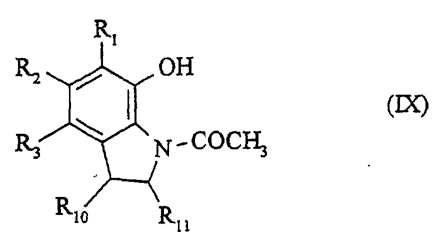 dans laquelle R₁₀ et R₁₁ représentent simultanément un hydrogène ou forment ensemble une liaison, R₁, R₂, R₃ ayant la même signification que dans la formule (I), qui, après déprotection de l'azote indolique, est traité par le 2,3-dibromopropionate d'éthyle en milieu basique, pour conduire au composé de formule (X/a) : 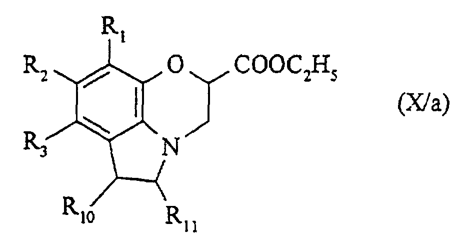 dans laquelle R₁, R₂, R₃, R₁₀, R₁₁ sont tels que définis précédemment,
que l'on peut faire réagir avec une base forte et un dérivé de formule (IV) :
R'₈-X (IV)
dans laquelle X représente un atome d'halogène et R'₈, différent d'un atome d'hydrogène, a la même signification que R₈ dans la formule (I),
pour conduire au composé de formule (X/b): 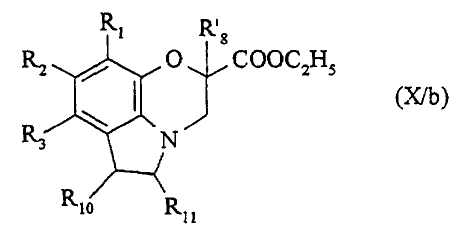 dans laquelle R₁, R₂, R₃, R'₈, R₁₀, R₁₁ sont tels que définis précédemment,
composés (X/a) et (X/b) qui représentent la totalité des composés de formule (X): 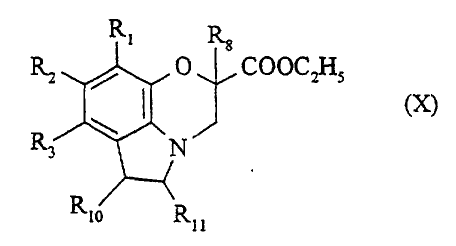 dans laquelle R₁, R₂, R₃, R₈, R₁₀ et R₁₁ sont tels que définis précédemment,
que l'on traite, après homologation éventuelle d'un carbone de la chaîne portant la fonction ester, par l'éthylènediamine, pour conduire au composé de formule (I/c) : 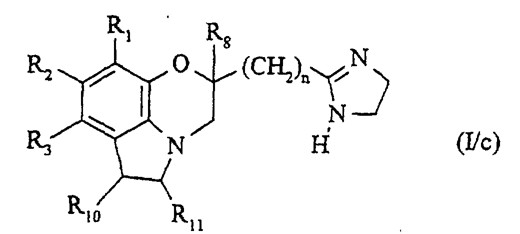 cas particulier des composés de formule (I) pour lequel R₁, R₂, R₃, R₈, R₁₀, R₁₁ et n sont tels que définis précédemment,
composé (I/c) :
- que l'on purifie éventuellement selon une technique classique de purification,
- dont on sépare éventuellement les isomères selon une technique classique de séparation,
- et que l'on transforme, le cas échéant, en son N-oxyde ou en son sel d'addition à un acide ou à une base pharmaceutiquement acceptable,
étant entendu que lorsque R₁₀ et R₁₁ représentent chacun un atome d'hydrogène, ils peuvent être transformés en une liaison par une méthode classique d'aromatisation, à tout moment du procédé.

16. Compositions pharmaceutiques contenant comme principe actif au moins un composé selon l'une quelconque des revendications 1 à 13, seul ou en combinaison avec un ou plusieurs excipents ou véhicules inertes, non toxiques, pharmaceutiquement acceptables.

17. Compositions pharmaceutiques selon la revendication 16 contenant au moins un principe actif selon l'une quelconque des revendications 1 à 8 utiles dans le traitement des maladies liées à des dérèglements du fonctionnement des récepteurs aux imidazolines comme les maladies cardiovasculaires et de l'hypertension artérielle, du diabète, de l'obésité, des désordres psychiatriques et neurologiques comme la dépression, la maladie de Parkinson, l'anorexie, la maladie d'Alzheimer.

18. Compositions pharmaceutiques selon la revendication 16 contenant un principe actif selon l'une quelconque des revendications 1 à 13 utiles dans le traitement des maladies cardiovasculaires et de l'hypertension artérielle.

## Claims

1. Compounds of formula (I): 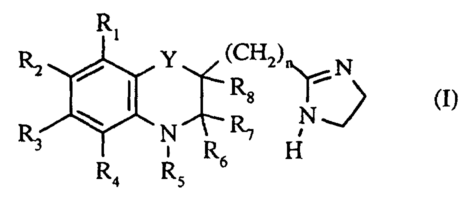 wherein:
• n is 0 or 1,
• Y represents an oxygen or sulphur atom or a CH₂ group,
• R₁, R₂, R₃ and R₄, which may be identical or different, represent a hydrogen or halogen atom or a linear or branched (C₁-C₆)alkyl, (C₃-C₇)cycloalkyl, linear or branched (C₁-C₆)alkoxy, hydroxy, linear or branched (C₁-C₆)hydroxyalkyl, linear or branched (C₁-C₆)trihaloalkyl, linear or branched (C₁-C₆)alkylcarbonyl, formyl, cyano, carboxy, linear or branched (C₁-C₆)alkoxycarbonyl, nitro or optionally substituted amino group,
or (R₁-R₂) or (R₂-R₃) or (R₃-R₄) form with the carbon atoms to which they are bonded an optionally substituted saturated or unsaturated 5- or 6-membered ring,
• R₅ represents a hydrogen atom, a (C₃-C₇)cycloalkyl group or a linear or branched (C₁-C₆)alkyl group optionally substituted by a group selected from (C₃-C₇)cycloalkyl and optionally substituted phenyl,
or, with R₄, forms a saturated or unsaturated 5-, 6- or 7-membered ring optionally substituted by one or more identical or different groups selected from linear or branched (C₁-C₆)alkyl, linear or branched (C₁-C₆)alkoxy, hydroxy, oxo and optionally substituted amino,
• R₆ and R₇ each represent a hydrogen atom or together form an oxo group,
• R₈ represents a halogen or hydrogen atom or a linear or branched (C₁-C₆)alkyl, linear or branched (C₂-C₆)alkenyl, hydroxy, linear or branched (C₁-C₆)alkoxy (optionally substituted by an amino, pyrrolyl or piperidinyl group), (C₃-C₇)cycloalkyloxy, optionally substituted phenyloxy or optionally substituted benzyloxy group;
or, with R₇, forms a bond,
with the proviso that :
- when n is 0, Y represents an oxygen atom and each of R₁, R₂, R₃, R₄, R₆, R₇ and R₈ represents a hydrogen atom, then R₅ is other than a hydrogen atom or a methyl, ethyl or benzyl group,
- when n is 0, Y represents a sulphur atom and each of R₁, R₂, R₃, R₄, R₆, R₇ and R₈ represents a hydrogen atom, then R₅ is other than a hydrogen atom,
- "optionally substituted amino group" is understood to mean substituted by one or two identical or different groups selected from linear or branched (C₁-C₆)alkyl, optionally substituted phenyl and optionally substituted benzyl,
- the expression "optionally substituted" applied to the groups phenyloxy, benzyloxy, phenyl and benzyl and to the expression "5- or 6-membered ring" means that those groups may be substituted by one or more identical or different substituents selected from halogen, linear or branched (C₁-C₆)alkyl, linear or branched (C₁-C₆)alkoxy, hydroxy and linear or branched (C₁-C₆)trihaloalkyl,
their N-oxides, their enantiomers and diastereoisomers, and addition salts thereof with a pharmaceutically acceptable acid or base.

2. Compounds of formula (I) according to claim 1 wherein n is 0, their N-oxides, their enantiomers and diastereoisomers, and addition salts thereof with a pharmaceutically acceptable acid or base.

3. Compounds of formula (I) according to claim 1 wherein Y represents an oxygen atom, their N-oxides, their enantiomers and diastereoisomers, and addition salts thereof with a pharmaceutically acceptable acid or base.

4. Compounds of formula (I) according to claim 1 wherein Y represents a sulphur atom, their N-oxides, their enantiomers and diastereoisomers, and addition salts thereof with a pharmaceutically acceptable acid or base.

5. Compounds of formula (I) according to claim 1 wherein each of R₆, R₇ and R₈ represents a hydrogen atom, their N-oxides, their enantiomers and diastereoisomers, and addition salts thereof with a pharmaceutically acceptable acid.

6. Compounds of formula (I) according to claim 1 wherein R₅ represents a linear or branched (C₁-C₆)alkyl group, their N-oxides, their enantiomers and diastereoisomers, and addition salts thereof with a pharmaceutically acceptable acid.

7. Compounds of formula (I) according to claim 1 wherein three of the groups R₁, R₂, R₃ and R₄ are identical and each of the three represents a hydrogen atom, the remaining group being as defined for formula (I), their N-oxides, their enantiomers and diastereoisomers, and addition salts thereof with a pharmaceutically acceptable acid.

8. Compounds of formula (I) according to claim 1 wherein (R₁-R₂) or (R₂-R₃) or (R₃-R₄) form with the carbon atoms to which they are bonded an optionally substituted saturated or unsaturated 5- or 6-membered ring, their N-oxides, their enantiomers and diastereoisomers, and addition salts thereof with a pharmaceutically acceptable acid.

9. Compounds of formula (I) according to claim 1 wherein R₄ and R₅ together form a saturated or unsaturated 5-, 6- or 7-membered ring optionally substituted by one or more identical or different groups selected from linear or branched (C₁-C₆)alkyl, linear or branched (C₁-C₆)alkoxy, hydroxy, oxo and optionally substituted amino; their N-oxides, their enantiomers and diastereoisomers, and addition salts thereof with a pharmaceutically acceptable acid or base.

10. Compounds of formula (I) according to claim 1 wherein Y represents an oxygen atom, n is 0, each of R₆, R₇ and R₈ represents a hydrogen atom, R₁, R₂, R₃ and R₄, which may be identical or different, represent a hydrogen or halogen atom or a linear or branched (C₁-C₆)alkyl, (C₃-C₇)cycloalkyl, linear or branched (C₁-C₆)alkoxy, hydroxy, linear or branched (C₁-C₆)hydroxyalkyl, linear or branched (C₁-C₆)-trihaloalkyl, linear or branched (C₁-C₆)alkylcarbonyl, formyl, cyano, carboxy, linear or branched (C₁-C₆)alkoxycarbonyl, nitro or optionally substituted amino group, or (R₁-R₂) or (R₂-R₃) or (R₃-R₄) form with the carbon atoms to which they are bonded an optionally substituted saturated or unsaturated 5- or 6-membered ring, whilst R₅ represents a linear or branched (C₁-C₆)alkyl group or, with R₄, forms a saturated or unsaturated 5-, 6- or 7-membered ring; their N-oxides, their enantiomers and diastereoisomers, and addition salts thereof with a pharmaceutically acceptable acid or base.

11. Compounds of formula (I) according to claim 1 which are :
- 2-(4,5-dihydro-1*H*-2-imidazolyl)-4,6-dimethyl-3,4-dihydro-2*H*-1,4-benzoxazine,
- 2-(4,5-dihydro- 1*H*-2-imidazolyl)-4,7-dimethyl-3,4-dihydro-2*H*-1,4-benzoxazine,
- 2-(4,5-dihydro-1*H*-2-imidazolyl)-4,8-dimethyl-3,4-dihydro-2H-1,4-benzoxazine,
their N-oxides, their enantiomers and diastereoisomers, and addition salts thereof with a pharmaceutically acceptable acid.

12. Compound of formula (I) according to claim 1 which is 6-chloro-2-(4,5-dihydro-1*H*-2-imidazolyl)-4-methyl-3,4-dihydro-2*H*-1,4-benzoxazine, its N-oxides, its enantiomers and diastereoisomers, and addition salts thereof with a pharmaceutically acceptable acid.

13. Compounds of formula (I) according to claim 1 which are :
- 2-(4,5-dihydro-1*H*-2-imidazolyl)-2,3,5,6-tetrahydro[1,4]oxazino[2,3,4-*h*,*i*]indole,
- 2-(4,5-dihydro-1*H*-2-imidazolyl)-2,3-dihydro[1,4]oxazino[2,3,4-*h*,*i*]indole,
their N-oxides, their enantiomers and diastereoisomers, and addition salts thereof with a pharmaceutically acceptable acid.

14. Process for the preparation of compounds of formula (I) characterised in that:
• when, in the desired compound of formula (I), R₄ and R₅ do not together form a ring, there is used as starting material a compound of formula (II/a): 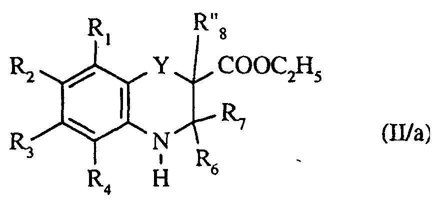 wherein R"₈ represents a hydrogen atom or, with R₇, forms a bond and Y, R₁, R₂, R₃, R₄, R₆ and R₇ are as defined for formula (I),
which is treated with either:
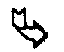 a compound of formula (III) :
R'₅-X (III)
wherein X represents a halogen atom and R'₅ represents a (C₃-C₇)-cycloalkyl group or a linear or branched (C₁-C₆)alkyl group optionally substituted by a (C₃-C₇)cycloalkyl or optionally substituted phenyl group,
to yield a compound of formula (II/b): 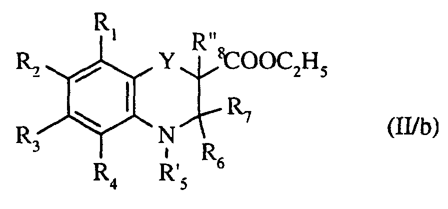 wherein Y, R₁, R₂, R₃, R₄, R'₅, R₆, R₇ and R"₈ are as defined above,
which may, when R"₈ represents a hydrogen atom, be subjected to the action of a halogenating agent, or to the action of a strong base followed by treatment with a compound of formula (IV) :
R'₈-X (IV)
wherein X represents a halogen atom and R'₈, which is other than a hydrogen atom, has the same meanings as R₈ for formula (I),
to yield a compound of formula (II/c): 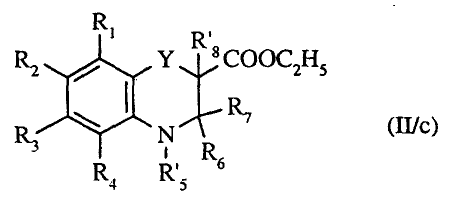 wherein Y, R₁, R₂, R₃, R₄, R'₅, R₆, R₇ and R'₈ are as defined above,
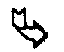 or, when, in the compound of formula (II/a) used, R"₈ represents a hydrogen atom, with a compound of formula (IV) as defined above, after treatment with a strong base,
to yield a compound of formula (II/d) : 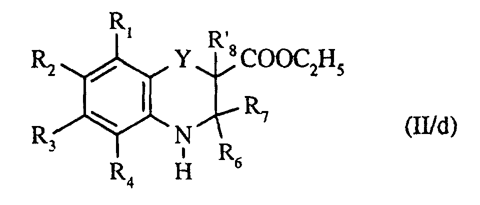 wherein Y, R₁, R₂, R₃, R₄, R₆, R₇ and R'₈ are as defined above,
which compounds (II/a), (II/b), (II/c) and (II/d) constitute the totality of the compounds of formula (II): 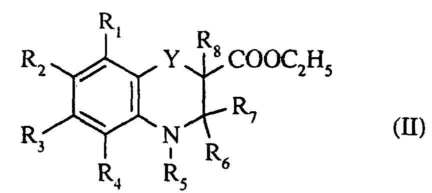 wherein Y, R₁, R₂, R₃, R₄, R₅, R₆, R₇ and R₈ are as defined above,
which is treated, optionally after homologisation of the chain that carries the ester function with a carbon atom, with ethylenediamine to yield a compound of formula (I/a) : 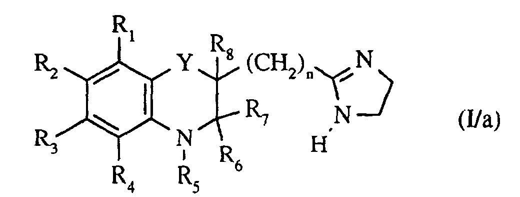 which is a particular case of the compounds of formula I wherein Y, R₁, R₂, R₃, R₄, R₅, R₆, R₇, R₈ and n have the same meanings as for formula (I), it being understood that R₄ and R₅ do not together form a ring,
or,
• when, in the desired compound of formula (I), R₄ and R₅ together form a ring, there is used as starting material a compound of formula (II/e) : 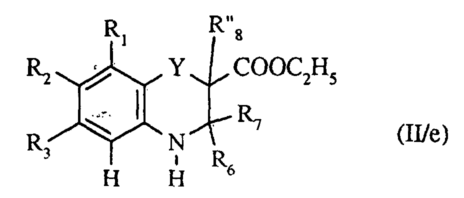 wherein R₁, R₂, R₃, R₆, R₇ and R"₈ are as defined above,
which is treated with a compound of formula (V) or (VI): 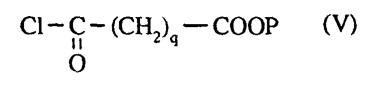 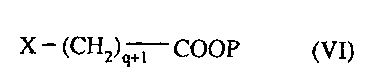 wherein X represents a halogen atom or a leaving group, P represents a group that masks the acid function, and q is an integer 0, 1 or 2,
to yield a compound of formula (VII): 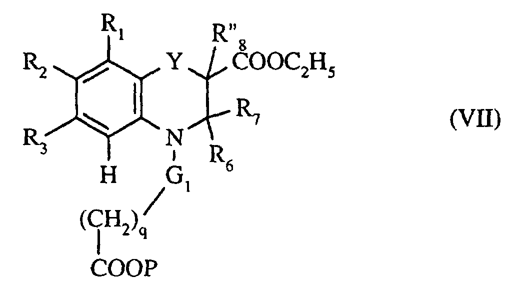 wherein G₁ represents a CH₂ or CO group, and q, P, Y, R₁, R₂, R₃, R₆, R₇ and R"₈ have the same meanings as above,
which, after freeing of the acid function and cyclisation, yields a compound of formula (VIII/a) : 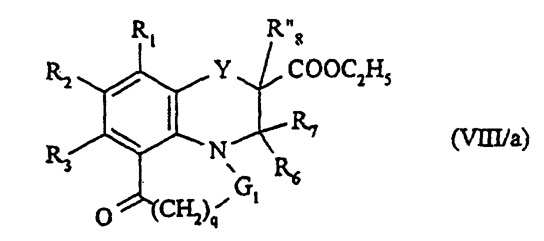 wherein Y, R₁, R₂, R₃, R₆, R₇, R"₈, G₁ and q have the same meanings as above,
which compound (VIII/a),
- can be converted, after treatment with an alkyl halide, to a compound of formula (VIII/b) : 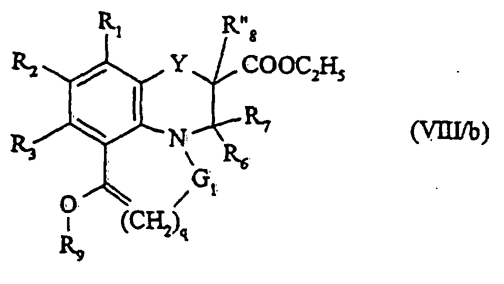 wherein R₉ represents a linear or branched (C₁-C₆)alkyl group, and Y, R₁, R₂, R₃, R₆, R₇, R"₈, G₁ and q have the same meanings as above,
- or can be reduced to yield a compound of formula (VIII/c): 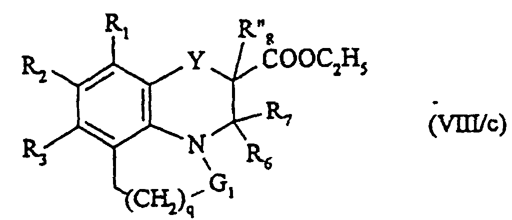 wherein Y, R₁, R₂, R₃, R₆, R₇, R"₈, G₁ and q are as defined above,
it being possible for each of the compounds (VIII/a), (VIII/b) and (VIII/c) to be treated, when that is compatible with the substituents present on the molecule, with a strong base and then with a compound of formula (IV) as defined above to yield a compound of formula (VIII/d) : 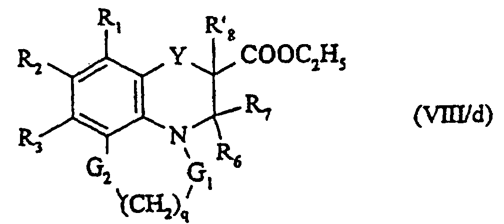 wherein G₂ represents a CH₂ or CO group, or, with the carbon atom of the adjacent methylene, forms a group 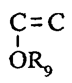 and Y, R₁, R₂, R₃, R₆, R₇, R'₈, R₉, G₁ and q are as defined above,
which compounds (VIII/a), (VIII/b), (VIII/c) and (VIII/d) constitute the totality of the compounds of formula (VIII): 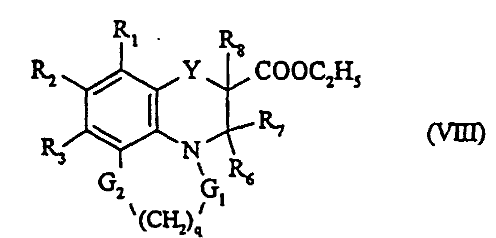 wherein R₁, R₂, R₃, R₆, R₇, R₈, G₁, G₂ and q are as defined above,
and which are treated, optionally after homologisation of the chain that carries the ester function with a carbon atom, with ethylenediamine to yield a compound of formula (I/b) : 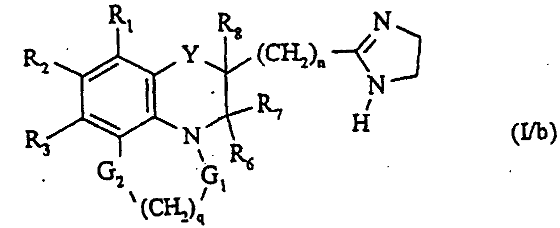 which is a particular case of the compounds of formula (I) wherein Y, R₁, R₂, R₃, R₆, R₇, R₈, G₁, G₂, n and q are as defined above,
which compounds (I/a) and (I/b):
- are optionally purified in accordance with a conventional purification technique,
- optionally separated into their isomers in accordance with a conventional separation technique, and
- converted, where appropriate, into their N-oxides or into addition salts thereof with a pharmaceutically acceptable acid or base.

15. Process for the preparation of compounds of formula (I) characterised in that there is used as starting material a compound of formula (IX): 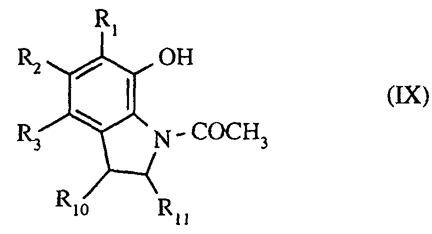 wherein R₁₀ and R₁₁ simultaneously represent a hydrogen atom or together form a bond, and R₁, R₂ and R₃ have the same meanings as for formula (I),
which, after deprotection of the indole nitrogen atom, is treated with ethyl 2,3-dibromopropionate in a basic medium to yield a compound of formula (X/a) : 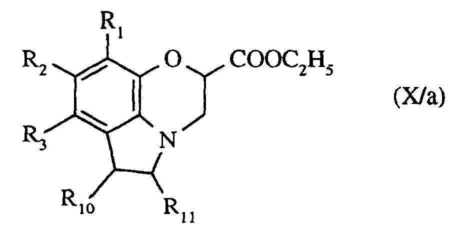 wherein R₁, R₂, R₃, R₁₀ and R₁₁ are as defined above,
which can be reacted with a strong base and a compound of formula (IV):
R'₈ - X (IV)
wherein X represents a halogen atom and R'₈, which is other than a hydrogen atom, has the same meanings as R₈ for formula (I),
to yield a compound of formula (X/b): 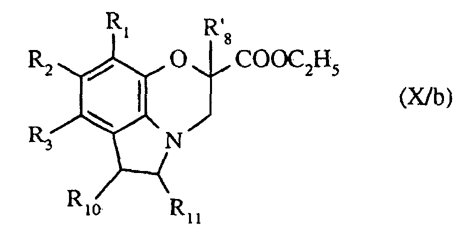 wherein R₁, R₂, R₃, R'₈, R₁₀ and R₁₁ are as defined above,
which compounds (X/a) and (X/b) constitute the totality of the compounds of formula (X): 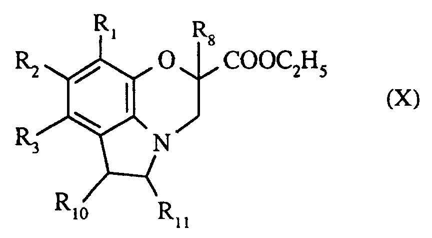 wherein R₁, R₂, R₃, R₈, R₁₀ and R₁₁ are as defined above,
which are treated, after optional homologisation of the chain that carries the ester function with a carbon atom, with ethylenediamine to yield a compound of formula (I/c): 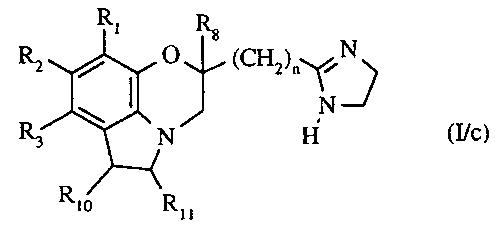 which is a particular case of the compounds of formula (I) wherein R₁, R₂, R₃, R₈, R₁₀, R₁₁ and n are as defined above,
which compound (I/c):
- is optionally purified in accordance with a conventional purification technique,
- optionally separated into its isomers in accordance with a conventional separation technique, and
- converted, where appropriate, into its N-oxide or into an addition salt thereof with a pharmaceutically acceptable acid or base,
it being understood that when each of R₁₀ and R₁₁ represents a hydrogen atom, they can be converted to a bond by a conventional aromatisation method at any moment in the process.

16. Pharmaceutical compositions comprising as active ingredient at least one compound according to any one of claims 1 to 13, on its own or in combination with one or more inert, non-toxic pharmaceutically acceptable excipients or carriers.

17. Pharmaceutical compositions according to claim 16 comprising at least one active ingredient according to any one of claims 1 to 8 for use in the treatment of diseases associated with disturbances in the functioning of the imidazoline receptors, such as cardiovascular diseases and arterial hypertension, diabetes, obesity, and psychiatric or neurological disorders, such as depression, Parkinson's disease, anorexia or Alzheimer's disease.

18. Pharmaceutical compositions according to claim 16 comprising an active ingredient according to any one of claims 1 to 13 for use in the treatment of cardiovascular diseases and arterial hypertension.

## Patentansprüche

1. Verbindungen der Formel (I): 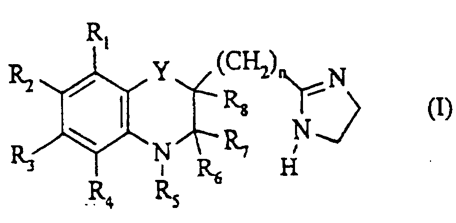 in der:
• n 0 oder 1 bedeutet,
• Y ein Sauerstoffatom, ein Schwefelatom oder eine Gruppe CH₂ darstellt,
• R₁, R₂, R₃, R₄, die gleichartig oder verschieden sind, Wasserstoffatome, Halogenatome, geradkettige oder verzweigte (C₁-C₆)-Alkylgruppen, (C₃-C₇)-Cycloalkylgruppen, geradkettige oder verzweigte (C₁-C₆)-Alkoxygruppen, Hydroxygruppen, geradkettige oder verzweigte (C₁-C₆)-Hydroxyalkylgruppen, geradkettige oder verzweigte (C₁-C₆)-Trihalogenalkylgruppen, geradkettige oder verzweigte (C₁-C₆)-Alkyl-carbonylgruppen, Formylgruppen, Cyanogruppen, Carboxygruppen, geradkettige oder verzweigte (C₁-C₆)-Alkoxy-carbonylgruppen, Nitrogruppen oder gegebenenfalls substituierte Aminogruppen bedeuten,
oder (R₁-R₂) oder (R₂-R₃) oder (R₃-R₄) zusammen mit den Kohlenstoffatomen, an die sie gebunden sind, einen gesättigten oder ungesättigten, gegebenenfalls substituierten Ring mit 5 oder 6 Kettengliedern bilden,
• R₅ ein Wasserstoffatom, eine (C₃-C₇)-Cycloalkylgruppe oder eine geradkettige oder verzweigte (C₁-C₆)-Alkylgruppe, die gegebenenfalls durch eine Gruppe ausgewählt aus (C₃-C₇)-Cycloalkyl und gegebenenfalls substituiertem Phenyl substituiert ist, bedeutet,
oder mit R₄ einen gesättigten oder ungesättigten, gegebenenfalls mit einer oder mehreren gleichartigen oder verschiedenen Gruppen ausgewählt aus geradkettigem oder verzweigtem (C₁-C₆)-Alkyl, geradkettigem oder verzweigtem (C₁-C₆)-Alkoxy, Hydroxy, Oxo und gegebenenfalls substituiertem Amino substituierten Ring mit 5, 6 oder 7 Kettengliedern bildet,
• R₆ und R₇ jeweils ein Wasserstoffatom bedeuten oder gemeinsam eine Oxogruppe bilden,
• R₈ ein Halogenatom, ein Wasserstoffatom oder eine geradkettige oder verzweigte (C₁-C₆)-Alkylgruppe, geradkettige oder verzweigte (C₂-C₆)-Alkenylgruppe, Hydroxygruppe, geradkettige oder verzweigte (gegebenenfalls durch eine Aminogruppe, Pyrrolylgruppe oder Piperidinylgruppe substituierte) (C₁-C₆)-Alkoxygruppe, (C₃-C₇)-Cycloalkoxygruppe, gegebenenfalls substituierte Phenyloxygruppe oder gegebenenfalls substituierte Benzyloxygruppe darstellt,
oder mit R₇ eine Bindung bildet,
mit der Maßgabe, daß:
- wenn n 0 ist, Y ein Sauerstoffatom darstellt und R₁, R₂, R₃, R₄, R₆, R₇ und R₈ jeweils ein Wasserstoffatom bedeuten, R₅ von einem Wasserstoffatom, einer Methylgruppe, einer Ethylgruppe oder einer Benzylgruppe verschieden ist,
- wenn n 0 ist, Y ein Schwefelatom darstellt und R₁, R₂, R₃, R₄, R₆, R₇ und R₈ jeweils ein Wasserstoffatom bedeuten, R₅ von einem Wasserstoffatom verschieden ist,
- unter einer gegebenenfalls substituierten Aminogruppe eine Substitution durch eine oder zwei gleichartige oder verschiedene Gruppen ausgewählt aus geradkettigem oder verzweigtem (C₁-C₆)-Alkyl, gegebenenfalls substituiertem Phenyl und gegebenenfalls substituiertem Benzyl zu verstehen ist,
- die Begriffe gegebenenfalls substituiert in bezug auf Phenyloxy-, Benzyloxy-, Phenyl- und Benzylgruppen sowie bezüglich des Begriffs "Ring mit 5 oder 6 Kettengliedern" bedeuten, daß diese Gruppen durch eine oder mehrere gleichartige oder verschiedene Substituenten ausgewählt aus Halogen, geradkettigem oder verzweigtem (C₁-C₆)-Alkyl, geradkettigem oder verzweigtem (C₁-C₆)-Alkoxy, Hydroxy und geradkettigem oder verzweigtem (C₁-C₆)-Trihalogenalkyl substituiert sein können,
deren N-Oxide, deren Enantiomere, Diastereoisomere sowie deren Additionssalze mit einer pharmazeutisch annehmbaren Säure oder Base.

2. Verbindungen der Formel (I) nach Anspruch 1, worin n 0 bedeutet, deren N-Oxide, deren Enantiomere, Diastereoisomere sowie deren Additionssalze mit einer pharmazeutisch annehmbaren Säure oder Base.

3. Verbindungen der Formel (I) nach Anspruch 1, worin Y ein Sauerstoffatom bedeutet, deren N-Oxide, deren Enantiomere, Diastereoisomere sowie deren Additionssalze mit einer pharmazeutisch annehmbaren Säure oder Base.

4. Verbindungen der Formel (I) nach Anspruch 1, worin Y ein Schwefelatom bedeutet, deren N-Oxide, deren Enantiomere, Diastereoisomere sowie deren Additionssalze mit einer pharmazeutisch annehmbaren Säure oder Base.

5. Verbindungen der Formel (I) nach Anspruch 1, worin R₆, R₇ und R₈ jeweils ein Wasserstoffatom bedeuten, deren N-Oxide, deren Enantiomere, Diastereoisomere sowie deren Additionssalze mit einer pharmazeutisch annehmbaren Säure.

6. Verbindungen der Formel (I) nach Anspruch 1, worin R₅ eine geradkettige oder verzweigte (C₁-C₆)-Alkylgruppe darstellt, deren N-Oxide, deren Enantiomere, Diastereoisomere sowie deren Additionssalze mit einer pharmazeutisch annehmbaren Säure.

7. Verbindungen der Formel (I) nach Anspruch 1, worin drei der Gruppen R₁, R₂, R₃ und R₄ identisch sind und jeweils ein Wasserstoffatom bedeuten, während die verbleibende Gruppe die bezüglich der Formel (I) angegebenen Bedeutungen besitzt, deren N-Oxide, deren Enantiomere, Diastereoisomere sowie deren Additionssalze mit einer pharmazeutisch annehmbaren Säure.

8. Verbindungen der Formel (I) nach Anspruch 1, worin (R₁-R₂) oder (R₂-R₃) oder (R₃-R₄) mit den Kohlenstoffatomen, an die sie gebunden sind, einen gesättigten oder ungesättigten, gegebenenfalls substituierten Ring mit 5 oder 6 Kettengliedern bilden, deren N-Oxide, deren Enantiomere, Diastereoisomere sowie deren Additionssalze mit einer pharmazeutisch annehmbaren Säure.

9. Verbindungen der Formel (I) nach Anspruch 1, worin R₄ und R₅ gemeinsam einen gesättigten oder ungesättigten, gegebenenfalls durch eine oder mehrere gleichartige oder verschiedene Gruppen ausgewählt aus geradkettigem oder verzweigtem (C₁-C₆)-Alkyl, geradkettigem oder verzweigtem (C₁-C₆)-Alkoxy, Hydroxy, Oxo und gegebenenfalls substituiertem Amino substituierten Ring mit 5, 6 oder 7 Kettengliedern bilden, deren N-Oxide, deren Enantiomere, Diastereoisomere sowie deren Additionssalze mit einer pharmazeutisch annehmbaren Säure oder Base.

10. Verbindungen der Formel (I) nach Anspruch 1, worin Y ein Sauerstoffatom darstellt, n den Wert 0 besitzt, R₆, R₇ und R₈ jeweils ein Wasserstoffatom darstellen, R₁, R₂, R₃ und R₄, die gleichartig oder verschieden sind, Wasserstoffatome, Halogenatome, geradkettige oder verzweigte (C₁-C₆)-Alkylgruppen, (C₃-C₇)-Cycloalkylgruppen, geradkettige oder verzweigte (C₁-C₆)-Alkoxygruppen, Hydroxygruppen, geradkettige oder verzweigte (C₁-C₆₎-Hydroxyalkylgruppen, geradkettige oder verzweigte (C₁-C₆)-Trihalogenalkylgruppen, geradkettige oder verzweigte (C₁-C₆)-Alkyl-carbonylgruppen, Formylgruppen, Cyanogruppen, Carboxygruppen, geradkettige oder verzweigte (C₁-C₆)-Alkoxy-carbonylgruppen, Nitrogruppen und gegebenenfalls substituierte Aminogruppen bedeuten, oder (R₁-R₂) oder (R₂-R₃) oder (R₃-R₄) zusammen mit den Kohlenstoffatomen, an die sie gebunden sind, einen gesättigten oder ungesättigten, gegebenenfalls substituierten Ring mit 5 oder 6 Kettengliedern bilden, während R₅ eine geradkettige oder verzweigte (C₁-C₆)-Alkylgruppe darstellt oder zusammen mit R₄ einen gesättigten oder ungesättigten Ring mit 5, 6 oder 7 Kettengliedern bildet, deren N-Oxide, deren Enantiomere, Diastereoisomere sowie deren Additionssalze mit einer pharmazeutisch annehmbaren Säure oder Base.

11. Verbindungen der Formel (I) nach Anspruch 1, nämlich:
- 2-(4,5-Dihydro-1*H*-2-imidazolyl)-4,6-dimethyl-3,4-dihydro-2*H*-1,4-benzoxazin,
- 2-(4,5-Dihydro-1*H*-2-imidazolyl)-4,7-dimethyl-3,4-dihydro-2*H*-1,4-benzoxazin,
- 2-(4,5-Dihydro-1*H*-2-imidazolyl)-4,8-dimethyl-3,4-dihydro-2*H*-1,4-benzoxazin,
deren N-Oxide, deren Enantiomere, Diastereoisomere sowie deren Additionssalze mit einer pharmazeutisch annehmbaren Säure.

12. Verbindung der Formel (I) nach Anspruch 1, nämlich 6-Chlor-2-(4,5-dihydro-1*H*-2-imidazolyl)-4-methyl-3,4-dihydro-2*H*-1,4-benzoxazin, dessen N-Oxide, dessen Enantiomere, Diastereoisomere sowie dessen Additionssalze mit einer pharmazeutisch annehmbaren Säure.

13. Verbindungen der Formel (I) nach Anspruch 1, nämlich:
- 2-(4,5-Dihydro-1*H*-2-imidazolyl)-2,3,5,6-tetrahydro[1,4]oxazino[2,3,4-*h*,*i*]indol,
- 2-(4,5-Dihydro-1*H*-2-imidazolyl)-2,3-dihydro[1,4]oxazino[2,3,4-*h*,*i*]indol,
deren N-Oxide, deren Enantiomere, Diastereoisomere sowie deren Additionssalze mit einer pharmazeutisch annehmbaren Säure.

14. Verfahren zur Herstellung der Verbindungen der Formel (I), dadurch gekennzeichnet, daß:
• wenn in der herzustellenden Verbindung der Forme (I) R₄ und R₅ nicht gemeinsam einen Ring bilden, man als Ausgangsmaterial eine Verbindung der Formel (II/a) verwendet: 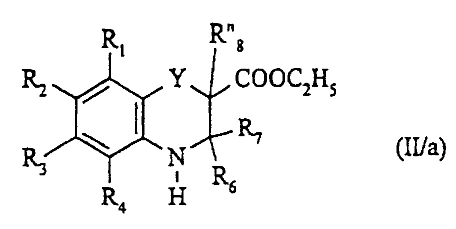 in der R"₈ ein Wasserstoffatom darstellt oder mit R₇ eine Bindung bildet und Y, R₁, R₂, R₃, R₄, R₆ und R₇ die bezüglich der Formel (I) angegebenen Bedeutungen besitzen,
welche man
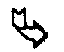 entweder mit einem Derivat der Formel (III) behandelt:
R'₅ - X (III)
in der X ein Halogenatom darstellt und R'₅ eine (C₃-C₇)-Cycloalkylgruppe oder eine geradkettige oder verzweigte, gegebenenfalls durch eine (C₃-C₇)-Cycloalkylgruppe oder eine gegebenenfalls substituierte Phenylgruppe substituierte (C₁-C₆)-Alkylgruppe darstellt,
zur Bildung einer Verbindung der Formel (II/b): 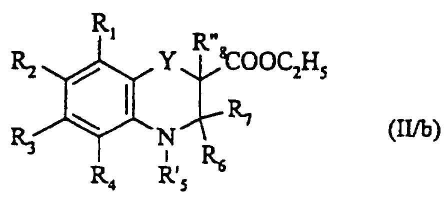 in der Y, R₁, R₂, R₃, R₄, R'₅, R₆, R₇ und R"₈ die oben angegebenen Bedeutungen besitzen,
welche, wenn R"₈ ein Wasserstoffatom bedeutet, der Einwirkung eines Halogenierungsmittels unterworfen werden kann oder der Einwirkung einer starken Base, gefolgt von einer Behandlung mit einem Derivat der Formel (IV):
R'₈-X (IV)
in der X ein Halogenatom darstellt und R'₈ von einem Wasserstoffatom verschieden ist und die gleichen Bedeutungen besitzt wie R₈ bezüglich der Formel (I),
zur Bildung einer Verbindung der Formel (II/c): 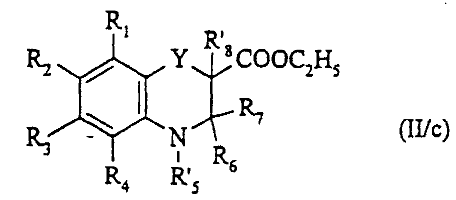 in der Y, R₁, R₂, R₃, R₄, R'₅, R₆ und R₇ und R'₈ die oben angegebenen Bedeutungen besitzen,
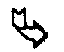 oder, wenn in der verwendeten Verbindung der Formel (II/a) R"₈ ein Wasserstoffatom bedeutet, nach einer Behandlung mit einer starken Base mit einem Derivat der Formel (IV) behandelt,
zur Bildung einer Verbindung der Formel (II/d): 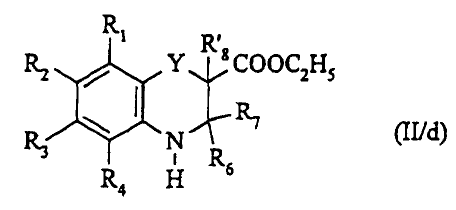 in der Y, R₁, R₂, R₃, R₄, R₆, R₇, und R'₈ die oben angegebenen Bedeutungen besitzen,
welche Verbindungen (II/a), (II/b), (II/c) und (II/d), welche die Gesamtheit der Verbindungen der Formel (II) bilden: 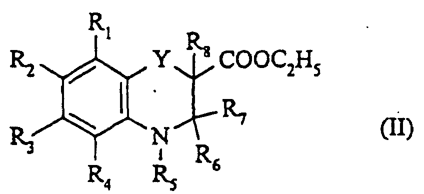 in der Y, R₁, R₂, R₃, R₄, R₅, R₆, R₇ und R₈ die oben angegebenen Bedeutungen besitzen,
man, gegebenenfalls nach der Homologierung eines Kohlenstoffatoms der die Esterfunktion tragenden Kette, mit Ethylendiamin behandelt zur Bildung einer Verbindung der Formel (I/a): 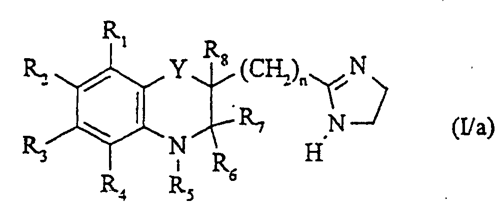 einem Sonderfall der Verbindungen der Formel I, worin Y, R₁, R₂, R₃, R₄, R₅, R₆, R₇, R₈ und n die bezüglich der Formel (I) angegebenen Bedeutungen besitzen, mit der Maßgabe, daß R₄ und R₅ nicht gemeinsam einen Ring bilden,
oder
• wenn in der herzustellenden Verbindung der Formel (I) R₄ und R₅ gemeinsam einen Ring bilden, man als Ausgangsmaterial eine Verbindung der Formel (II/e) verwendet: 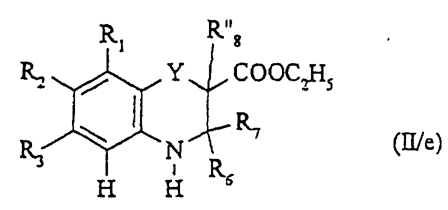 in der R₁, R₂, R₃, R₆, R₇ und R"₈ die oben angegebenen Bedeutungen besitzen,
welche man mit einem Derivat der Formel (V) oder (VI) behandelt: 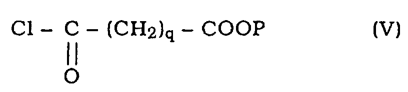
X - (CH₂)_{q+1} - COOP (VII)
in denen X ein Halogenatom oder eine austretende Gruppe, P eine maskierte Gruppe für die Säurefunktion und q eine ganze Zahl mit einem Wert von 0, 1 oder 2 bedeuten,
zur Bildung einer Verbindung der Formel (VII): 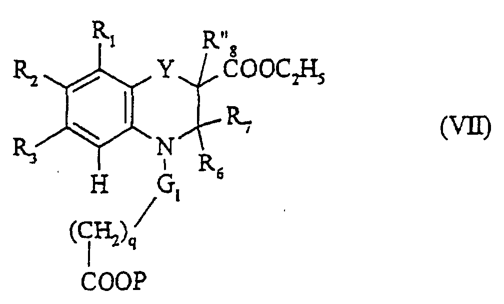 in der G₁ eine Gruppe CH₂ oder CO darstellt und q, P, Y, R₁, R₂, R₃, R₆, R₇ und R"₈ die oben angegebenen Bedeutungen besitzen,
welche, nach der Freisetzung der Säurefunktion und der Cyclisierung zu der Verbindung der Formel (VIII/a) führt: 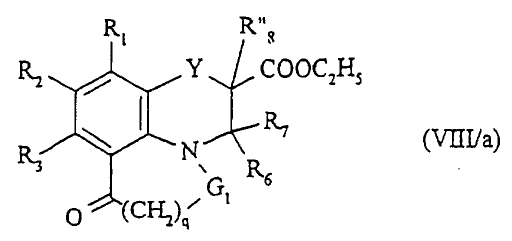 in der Y, R₁, R₂, R₃, R₆, R₇, R"₈, G₁ und q die oben angegebenen Bedeutungen besitzen,
welche Verbindung (VIII/a)
- nach der Behandlung mit einem Alkylhalogenid in eine Verbindung der Formel (VIII/b) umgewandelt werden kann: 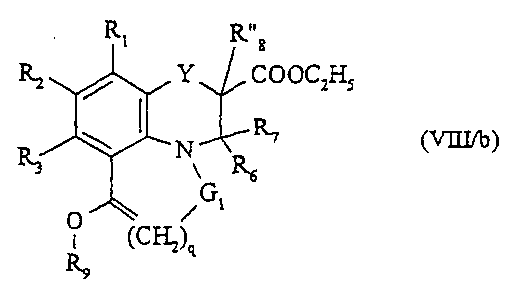 in der R₉ eine geradkettige oder verzweigte (C₁-C₆)-Alkylgruppe darstellt und Y, R₁, R₂, R₃, R₅, R₇, R"₈, G₁ und q die oben angegebenen Bedeutungen besitzen,
- oder reduziert werden kann zur Bildung einer Verbindung der Formel (VIII/c): 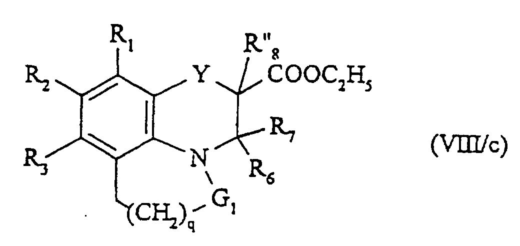 in der Y, R₁, R₂, R₃, R₆, R₇, R"₈, G₁ und q die oben angegebenen Bedeutungen besitzen,
wobei jede der Verbindungen (VIII/a), (VIII/b) und (VIII/c), wenn dies mit den an dem Molekül vorhandenen Substituenten verträglich ist, mit einer starken Base und dann mit einem Derivat der oben definierten Formel (IV) behandelt werden können zur Bildung einer Verbindung der Formel (VIII/d): 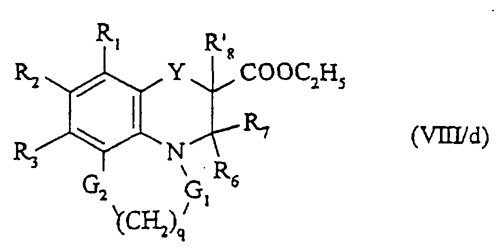 in der G₂ eine Gruppe CH₂ oder CO darstellt oder gemeinsam mit dem benachbarten Methylenkohlenstoff eine Gruppe der Formel bildet: 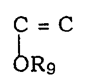 und Y, R₁, R₂, R₃, R₆, R₇, R'₈, R₉, G₁ und q die oben angegebenen Bedeutungen besitzen,
welche Verbindungen der (VIII/a), (VIII/b), (VIII/c) und (VIII/d) die Gesamtheit der Verbindungen der Formel (VIII) bilden: 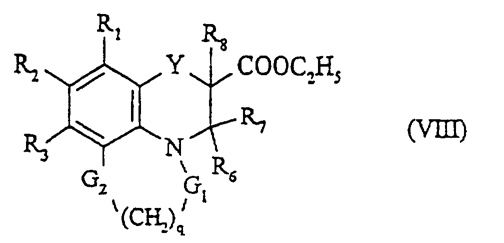 in der R₁, R₂, R₃, R₆, R₇, R₈, G₁, G₂ und q die oben angegebenen Bedeutungen besitzen,
und die, gegebenenfalls nach der Homologierung eines Kohlenstoffatoms der die Esterfunktion tragenden Kette, mit Ethylendiamin behandelt werden zur Bildung einer Verbindung der Formel (I/b): 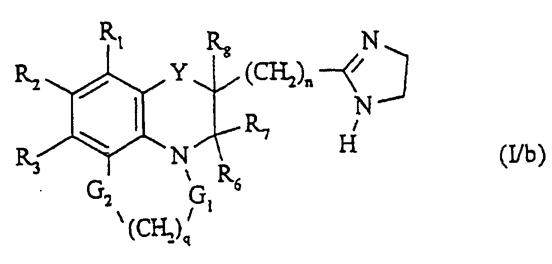 einem Sonderfall der Verbindungen der Formel (I), worin Y, R₁, R₂, R₃, R₆, R₇, R₈, G₁, G₂, n und q die oben angegebenen Bedeutungen besitzen,
welche Verbindungen (I/a) und (I/b) man:
- gegebenenfalls mit Hilfe einer klassischen Reinigungsmethode reinigt,
- gegebenenfalls mit Hilfe einer klassischen Trennmethode in ihre Isomeren auftrennt,
- und gegebenenfalls in ihre N-Oxide oder ihre Additionssalze mit einer pharmazeutisch annehmbaren Säure oder Base umwandelt.

15. Verfahren zur Herstellung der Verbindungen der Formel (I), dadurch gekennzeichnet, daß man als Ausgangsmaterial eine Verbindung der Formel (IX) verwendet: 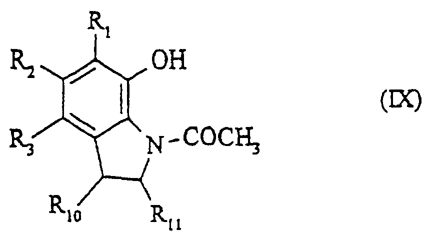 in der R₁₀ und R₁₁ gleichzeitig Wasserstoff bedeuten oder gemeinsam eine Bindung bilden und R₁, R₂ und R₃ die bezüglich der Formel (I) angegebenen Bedeutungen besitzen,
welche nach der Abspaltung der Schutzgruppe von dem Indolstickstoffatom mit 2,3-Dibrompropionsäuremethylester in basischem Medium behandelt werden zur Bildung der Verbindung der Formel (X/a): 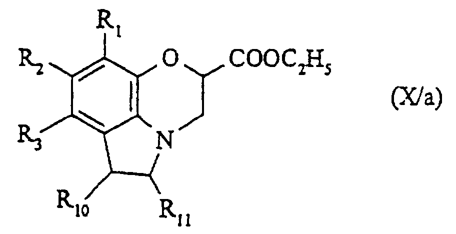 in der R₁, R₂, R₃, R₁₀ und R₁₁ die oben angegebenen Bedeutungen besitzen, welche man mit einer starken Base und einem Derivat der Formel (IV) umsetzt:
R'₈ - X (IV)
In der X ein Halogenatom darstellt und R'₈, welches von einem Wasserstoffatom verschieden ist, die gleichen Bedeutungen besitzt wie R₈ bezüglich der Formel (I),
zur Bildung der Verbindung der Formel (X/b): 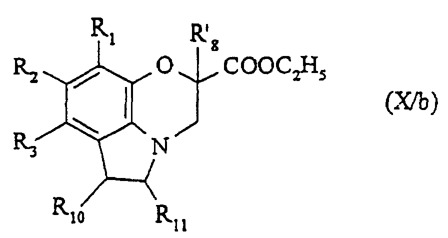 in der R₁, R₂, R₃, R'₈, R₁₀ und R₁₁ die oben angegebenen Bedeutungen besitzen,
welche Verbindungen (X/a) und (X/b) die Gesamtheit der Verbindungen der Formel (X) bilden: 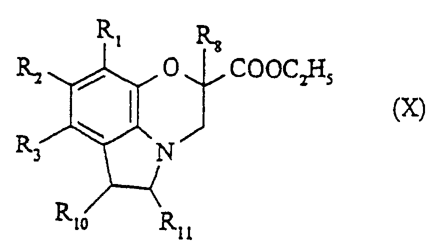 in der R₁, R_{2,} R₃, R₈, R₁₀ und R₁₁ die oben angegebenen Bedeutungen besitzen,
welche man, nach einer eventuellen Homologierung eines Kohlenstoffatoms der die Esterfunktion tragenden Kette, mit Ethylendiamin behandelt zur Bildung einer Verbindung der Formel (I/c): 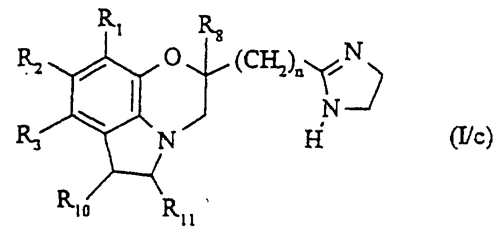 einem Sonderfall der Verbindungen der Formel (I), worin R₁, R₂, R₃, R₈, R₁₀, R₁₁ und n die oben angegebenen Bedeutungen besitzen,
welche Verbindung (I/c) man:
- gegebenenfalls mit Hilfe einer klassischen Reinigungsmethode reinigt,
- gegebenenfalls mit Hilfe einer klassischen Trennmethode in ihre Isomeren auftrennt,
- und gegebenenfalls in ihr N-Oxid oder ihr Additionssalz mit einer pharmazeutisch annehmbaren Säure oder Base umwandelt,
wobei es sich versteht, daß, wenn R₁₀ und R₁₁ jeweils ein Wasserstoffatom bedeuten, sie in irgendeinem Augenblick des Verfahrens mit Hilfe einer klassischen Aromatisierungsmethode in eine Bindung umgewandelt werden können.

16. Pharmazeutische Zubereitungen enthaltend als Wirkstoff mindestens eine Verbindung nach einem der Ansprüche 1 bis 13 allein oder in Kombination mit einem oder mehreren inerten, nichttoxischen, pharmazeutisch annehmbaren Trägermaterialien oder Bindemitteln.

17. Pharmazeutische Zubereitungen nach Anspruch 16 enthaltend mindestens einen Wirkstoff nach einem der Ansprüche 1 bis 8 zur Behandlung von Erkrankungen, die mit Störungen der Funktion von Imidazolin-Rezeptoren verknüpft sind, wie kardiovaskulären Krankheiten und arterieller Hypertonie, Diabetes, Fettsucht, psychiatrische und neurologische Störungen, wie Depressionen, Parkinsonsche Krankheit, Anorexie und Alzheimersche Krankheit.

18. Pharmazeutische Zubereitungen nach Anspruch 16 zur Behandlung von kardiovaskulären Erkrankungen und der arteriellen Hypertonie enthaltend einen Wirkstoff nach einem der Ansprüche 1 bis 13.
